# EUROPEAN PATENT APPLICATION

(11) **EP 4 203 378 A1**
(43) Date of publication of application: **28.06.2023**
(21) Application number: 22000262.0
(22) Date of filing: 19.12.2022
(51) Int. Cl.: H04L 9/00, G06Q 10/30, G06Q 10/0833, G06Q 50/04, G06Q 10/10, H04L 9/40

(54) **APPARATUS FOR GENERATING A DIGITAL ACCESS ELEMENT ASSOCIATED WITH A POLYSTYRENE COMPOSITION**

(30) Priority: 21.12.2021 EP 21216326; 21.12.2021 EP 21216327; 21.12.2021 EP 21216333; 21.12.2021 EP 21216270; 21.12.2021 EP 21216268; 21.12.2021 EP 21216269; 21.12.2021 EP 21216271; 21.12.2021 EP 21216286; 21.12.2021 EP 21216292; 04.04.2022 EP 22166573; 12.04.2022 EP 22167945; 10.05.2022 EP 22172619; 10.05.2022 EP 22172611; 10.05.2022 EP 22172615; 10.05.2022 EP 22172617; 10.05.2022 EP 22172609; 09.09.2022 EP 22194818; 09.09.2022 EP 22194793; 09.09.2022 EP 22194800; 09.09.2022 EP 22194808; 09.09.2022 EP 22194815; 14.10.2022 US 202263416091 P; 14.10.2022 EP 22201672; 18.10.2022 EP 22202183; 05.12.2022 EP 22211421
(71) Applicant: BASF SE, 67056 Ludwigshafen am Rhein (DE)
(72) Inventor: Decker, Andrea, 67056 Ludwigshafen am Rhein (DE); Klein, Marcel Dominik, 67056 Ludwigshafen am Rhein (DE); Kutscher, Stefanie, 67056 Ludwigshafen am Rhein (DE); Haverkemper, Gregor, 67056 Ludwigshafen am Rhein (DE); Ries, Klaus, 67056 Ludwigshafen am Rhein (DE); Hoeffken, Matthias Frithjof, 67056 Ludwigshafen am Rhein (DE); Schwabe, Henning, 67056 Ludwigshafen (DE); Wollny, Andreas, 67056 Ludwigshafen am Rhein (DE); Haardt, Dennis, 67056 Ludwigshafen am Rhein (DE)
(74) Representative: BASF IP Association

(57) **Abstract**

Disclosed is an apparatus for generating a digital access element associated with a polystyrene composition is presented, wherein the apparatus comprises a) a digital identifier providing unit configured to provide a digital identifier associated with the polystyrene composition and polystyrene composition data, and b) a digital access element generator configured to generate the digital access element based on the digital identifier and access data

## Description

### TECHNICAL FIELD

The present disclosure relates to an apparatus for generating a digital access element associated with a polystyrene composition, and to a corresponding method and computer program. The present disclosure further relates to the use of the generated digital access element, a polystyrene composition associated with the generated digital access element, a system including a polystyrene composition associated with the generated digital access element and a digital access element associated with the polystyrene composition and including a digital identifier and access data.

### TECHNICAL BACKGROUND

In the supply of chemical products multiple regulatory requirements need to be met, which differ depending on the chemical product. For instance, in automotive supply chains chemical companies provide standardized information using the International Material Data System (IMDS). Such system allows to collect data along the entire automotive supply chain. Participants in the automotive supply chain register with the IMDS service and maintain product entries in the central database as provided and hosted by a third-party provider.

Systems like IMDS are static regarding data, prone to error and cumbersome in handling or maintenance. Owing to the highly specific and centralized setup of such systems, exchange and sharing of chemicals data is laborious. Moreover, the centralized setup raises concerns regarding data security. Hence, there is a need to render chemical data exchange and sharing simpler and at the same time more secure. This need is felt particularly by members of supply chains involving polystyrene composition production, for example expanded polystyrene production and/or extruded polystyrene production, since these supply chains bridge a technically and logistically wide span from raw materials to the end customer and would therefore particularly benefit from means for a simple and secure data exchange.

### SUMMARY OF THE INVENTION

It is an object of the invention to provide a simpler and more secure way of exchanging data on polystyrene compositions, such as expanded polystyrene and/or extruded polystyrene.

In one aspect an apparatus for generating a digital access element associated with a polystyrene composition is presented, wherein the apparatus comprises a) a digital identifier providing unit configured to provide a digital identifier associated with the polystyrene composition and polystyrene composition data, and b) a digital access element generator configured to generate the digital access element based on the digital identifier and access data.

In one aspect an apparatus for generating a digital access element associated with a polystyrene composition is presented, the apparatus comprising: one or more computing nodes; and one or more computer-readable media having thereon computer-executable instructions that are structured such that, when executed by the one or more computing nodes, cause the apparatus to perform the following steps:
- receiving a request to provide a digital identifier associated with a data owner and polystyrene composition data, particularly wherein the data owner controls access to the polystyrene composition data, e.g. by a data consuming service
- in response to the request, providing the decentral identifier and generating the digital access element including the digital identifier and access data;
- providing the digital access element for access by a data consuming service under control or controlled by a data providing service associated with the data owner, particularly wherein the data providing service comprises computer-executable instructions for providing and/or processing polystyrene composition data associated with the data owner e.g. for accessing and/or processing by the data consuming service.

In one aspect an apparatus for generating a digital access element associated with a polystyrene composition is presented, the apparatus comprising: one or more computing nodes; and one or more computer-readable media having thereon computer-executable instructions that are structured such that, when executed by the one or more computing nodes, cause the apparatus to perform the following steps:
- providing a digital identifier associated with a data owner and polystyrene composition data, particularly wherein the data owner controls access to the polystyrene composition data, e.g. by a data consuming service
- generating the digital access element including the digital identifier and access data;
- providing the digital access element for access by a data consuming service under control or controlled by a data providing service associated with a data owner, particularly wherein the data providing service comprises computer-executable instructions for providing and/or processing polystyrene composition data associated with the data owner e.g. for accessing and/or processing by the data consuming service.

In a further aspect, the invention relates to a method, in particular to a computer-implemented method, for generating a digital access element associated with a polystyrene composition, wherein the method includes a) providing a digital identifier associated with the polystyrene composition and polystyrene composition data, and b) generating the digital access element based on the digital identifier and access data.

In another aspect disclosed is a computer-implemented method for generating a digital access element associated with a polystyrene composition, said digital access element including a digital identifier and access data, particularly a digital representation pointing to polystyrene composition data associated with the polystyrene composition, the method comprising the steps:
- receiving a request to provide the digital identifier associated with polystyrene composition data and a data owner, particularly wherein the data owner controls access to the polystyrene composition data, e.g. by a data consuming service;
- in response to the request, providing the decentral identifier and generating the digital access element including the digital identifier and the access data;
- providing the digital access element for access by the data consuming service controlled by or under control by a data providing service associated with the data owner, particularly wherein the data providing service comprises computer-executable instructions for providing and/or processing polystyrene composition data associated with the data owner e.g. for accessing and/or processing by the data consuming service.

In another aspect disclosed is a computer-implemented method for generating a digital access element associated with a polystyrene composition, said digital access element including a digital identifier and access data, particularly a digital representation pointing to polystyrene composition data associated with the polystyrene composition, the method comprising the steps:
- providing a digital identifier associated with a data owner and polystyrene composition data, particularly wherein the data owner controls access to the polystyrene composition data, e.g. by a data consuming service
- generating the digital access element including the digital identifier and access data;
- providing the digital access element for access by a data consuming service under control or controlled by a data providing service associated with a data owner, particularly wherein the data providing service comprises computer-executable instructions for providing and/or processing polystyrene composition data associated with the data owner e.g. for accessing and/or processing by the data consuming service.

In yet another aspect disclosed is a computer-implemented method for using a digital access element, preferably to further process the polystyrene composition associated with the digital access element, the method comprising the steps:
- receiving a request to access polystyrene composition data associated with a digital identifier of the digital access element as generated according to the methods disclosed herein or by the apparatuses disclosed herein;
- optionally authenticating and/or authorizing the request to access the polystyrene composition data;
- based on optionally the authentication and/or authorization, providing access to the polystyrene composition data associated with the digital identifier of the digital access element.

In a further aspect disclosed is a use of the digital access element as generated by the above apparatuses or according to the above methods during the manufacturing of products using a polystyrene composition associated with the digital access element.

In yet another aspect a polystyrene composition associated with the digital access element is disclosed, wherein the digital access element including the digital identifier and the access data is generated for the polystyrene composition according to the methods or by the apparatuses lined out herein.

In yet another aspect a system including a polystyrene composition associated with a digital access element is disclosed, wherein the digital access element including a digital identifier and access data is generated for the polystyrene composition according to the methods or by the apparatuses lined out herein.

In yet another aspect a digital access element including a digital identifier and access data is disclosed, wherein the digital access element is generated for the polystyrene composition according to the methods or by the apparatuses lined out herein.

In a further aspect disclosed is a computer program for generating a digital access element associated with a polystyrene composition, wherein the program comprises instructions which, when the program is executed by one or more computers, cause the one or more computers to execute the above methods. The one or more computers can realize a distributed computing environment in which the program can be executed.

Any disclosure and embodiments described herein relate to the methods, the apparatuses, the systems, the polystyrene compositions, the digital access elements, the uses, the computer elements lined out above or below and vice versa. The benefits provided by any of the embodiments and examples equally apply to all other embodiments and examples.

The methods, apparatuses, systems and computer elements disclosed herein provide an efficient, secure and robust way for sharing or exchanging data across different participant nodes in chemical value chains. Since a digital access element for a polystyrene composition is generated based on a digital identifier which is associated with the polystyrene composition and polystyrene composition data, and further based on access data, the digital access element allows for an indirect access to the polystyrene composition data, i.e. an access to the polystyrene composition data via the digital access element. Access to the digital access element itself can remain unrestricted while still allowing for controlled access to the polystyrene composition data. It has been found that such a digital access element allows to exchange polystyrene composition data in a simpler and more secure way.

The digital access element could be further defined as a digital data structure allowing third parties to access data on the polystyrene composition, wherein the access is usually conditional. The provided digital identifier may be understood as a unique digital reference to a digital identity or a digital twin representing a physical identity of the polystyrene composition, or as a unique digital name of the polystyrene composition, wherein the digital identity or digital twin representing the physical identity of the polystyrene composition may be given in terms of the data on the polystyrene composition, i.e. the machine polystyrene composition. Therefore, and since a passport usually allows to uniquely identify an individual, the digital access element may also be understood as a digital passport for the polystyrene composition or as a polystyrene composition passport. However, unlike ordinary digital passports which list all constituents of a material or even fully characterize the material, the digital access element preferably includes, if at all, only selected data on the polystyrene composition itself, but otherwise just access data allowing for access to further polystyrene composition data. This can increase data security. For instance, by virtue of digital access elements as described herein, the data owner of the polystyrene composition data may control access by participant nodes or data consuming services of the decentral network to the polystyrene composition data. At the same time, controlled data transparency is facilitated by providing means for accessing the data. The digital access element could even be publicly accessible.

The digital access element may allow to improve recycling process of materials being produced from the polystyrene composition. For instance, the digital access element may be used to retrieve polystyrene composition data which in turn may be used to generate recycling instructions or disposal instructions. The recycling instructions may comprise suitable recycling plants, suitable recycling operations.

### Embodiments

In the following, terminology as used herein and/or the technical field of the present disclosure will be outlined by ways of definitions and/or examples. Where examples are given, it is to be understood that the present disclosure is not limited to said examples.

The polystyrene composition may comprise a polystyrene and/or a copolymer produced from styrene or mixtures of polymers of this type. The polystyrene composition may further comprise at least one additive. The at least one additive may be selected from glass fiber, flame retardant, catalyst, blowing agent, colorant, and mixtures thereof.

In an example, the digital identifier is a decentralized identifier (DID) as described, for instance, in the W3C recommendation dated 19 July 2022. The digital access element may then be regarded as a DID document. The subject of the digital identifier, i.e. in that case of the DID, however, is then not any generic subject, but specifically a polystyrene composition. In another example, the digital identifier is a Universally Unique Identifier (UUID) such as, for instance, according to the UUIDv4 standard. The digital identifier may be associated with or include one or more identifier(s) used in a decentral network and allowing for data exchange via the decentral network. For instance, the digital identifier may be associated with one or more decentralized identifier(s) (DID(s), such as with one or more DID(s) of data sets contained in the digital twin of the polystyrene composition. In another instance, the digital identifier may be associated with one or more Universally Unique identifiers) (UUID(s)), such as with one or more UUID(s) of data sets contained in the digital twin of the polystyrene composition. In yet another instance, the digital identifier may be associated with one or more decentralized identifier(s) (DID(s) and one or more Universally Unique Identifier(s) (UUID(s)), such as with one or more DID(s) of data sets and one or more UUID(s) of data sets contained in the digital twin of the polystyrene composition. Data exchange may include discovery of the digital identifier(s) by participant nodes of the decentral network, authentication of participant nodes of the decentral network and/or authorization of data transfers via a peer-to-peer communication between participant nodes of the decentral network.

The digital identifier is associated with the polystyrene composition, and particularly with its digital and/or physical identity. Moreover, the digital identifier is associated with data on the polystyrene composition, i.e. polystyrene composition data. The digital identifier may be associated with an owner (e.g. data owner) of the polystyrene composition data. The digital identifier may be associated the polystyrene composition producer. The digital identifier may be associated with a machine, a system, or a device used for producing the polystyrene composition, or a collection of such machine(s), device(s) and/or system(s). In fact, the digital identifier may be a unique identifier uniquely associated with the polystyrene composition data and the owner of the polystyrene composition data. Via the digital identifier and its preferably unique association with the data owner and the polystyrene composition data, access to the polystyrene composition data may be controlled by the data owner. This contrasts with central authority schemes, where identifiers are provided by a central authority and access to data is controlled by such central authority. While digital identifiers based on which digital access elements shall be generated as described herein may also be issued by a central identity issuer, access to the polystyrene composition data shall at least not be controlled by this central identity issue. It may be preferred that the digital identifier based on which a digital access element is generated is issued by a decentral identity issuer. The term "decentral" refers in this context to the usage of the identifier in implementations as controlled by the data owner.

A data owner may be any entity generating data, particularly polystyrene composition data. Hence, by generating the data, an entity may become the owner of the generated data. The data, particularly the polystyrene composition data, may be generated by a third-party entity on behalf of the entity owning physical products from or for which data is generated. Generating data may refer to data acquisition, such as by measurements, but could also refer to a processing of data if the processing leads to new data, wherein "new data" may be characterized by new technical information as compared to the non-processed data. The data generating node may be coupled to the entity owning polystyrene compositions from or for which data, particularly, the polystyrene composition data, is generated. Instead of being derived from who generated the data, data ownership may also derive from ownership over a production producing the polystyrene composition. Hence, for instance, if the producer of the polystyrene composition does not acquire or otherwise generate the polystyrene composition data, but if instead a third-party entity acquires or otherwise generates the polystyrene composition data, the producer of polystyrene composition may be the owner of the polystyrene composition data. The polystyrene composition data may be accessible for the data owner. The data owner may hence directly or indirectly own the polystyrene composition data. The polystyrene composition data may be stored in a data base of or associated with the data owner. The polystyrene composition data may be stored in a data base accessible by the data owner. Via the digital identifier and its unique association with the data owner and polystyrene composition data, access to the polystyrene composition data may be controlled by the data owner. For instance, the data owner may control access to the polystyrene composition data, for example via a data providing service of the data owner. The polystyrene composition data may be associated with the data owner. The data owner may be the owner of the polystyrene composition data or the polystyrene composition data owner. In this sense, the data owner is to be construed broadly as the entity having access to the polystyrene composition data and controlling access by data consuming services of the decentral network to the polystyrene composition data.

The access data may refer to any data for accessing the polystyrene composition data. The polystyrene composition data may be associated with the polystyrene composition. The access data may be indispensable, i.e. strictly necessary for accessing the polystyrene composition data. Alternatively, the access data may be suitable for accessing the polystyrene composition data, while the polystyrene composition data could also be accessed in different ways, without the access data based on which the digital access element is generated. Access data may include an endpoint for data exchange or sharing (resource endpoint) or an endpoint for service interaction (service endpoint), that is uniquely identified via a communication protocol. Access data may include authorization schemes and/or cryptographic information. For instance, the access data may include a public key, such as a public key needed for decrypting the polystyrene composition data. The access data may be uniquely associated with the digital identifier. The access data may be provided to the data consuming service. The access data may be provided by a decentral network database, a database associated with the data consuming service, the data providing service associated with the data owner or combinations thereof.

Recycled content data may comprise any data related to the recyclate content used for providing or producing the polystyrene composition. For instance, the recyclate content of an engine coolant may stem from using recycled ethylene glycol.

Emission data may comprise any data related to environmental footprint. The environmental footprint may refer to the polystyrene composition and its associated environmental footprint. The environmental footprint may be entity specific. For instance, the environmental footprint may relate to the polystyrene composition, the company producing the polystyrene composition, a process such as a manufacturing process, combinations thereof or additional entity-specific relations. Emission data may include data relating to the carbon footprint of the polystyrene composition or a Product Carbon Footprint (PCF). Emission data may include data relating to greenhouse gas emissions e.g. released in production of the polystyrene composition. Emission data may include data related to greenhouse gas emissions. Greenhouse gas emissions may include emissions such as carbon dioxide (CO₂) emission, methane (CH₄) emission, nitrous oxide (N₂O) emission, hydrofluorocarbons (HFCs) emission, perfluorocarbons (PFCs) emission, sulphurhexafluoride (SF₆) emission, nitrogen trifluoride (NF₃) emission, combinations thereof and additional emissions.

Emission data may include data related to greenhouse gas emissions of an entities or companies own operations (production, power plants and waste incineration). Scope 2 may comprise emissions from energy production which is sourced externally. Scope 3 may comprise all other emissions along the value chain. Specifically, this may include the greenhouse gas emissions of raw materials obtained from suppliers. Product Carbon Footprint (PCF) may sum up greenhouse gas emissions and removals from the consecutive and interlinked process steps related to a particular polystyrene composition. Cradle-to-gate PCF may sum up greenhouse gas emissions based on selected process steps: e.g. from the extraction of resources up to the factory gate where the product leaves the company. Such PCFs may be called partial PCFs. In order to achieve such summation, each company providing any products may provide the scope 1 and scope 2 contributions to the PCF for each of its products.

It shall be understood that the apparatus for generating the digital access element associated with the polystyrene composition may comprise one or more computing nodes and one or more computer-readable media having thereon computer-executable instructions which, when executed by the one or more computing nodes, cause any unit of the apparatus, particularly the digital identifier providing unit and the digital access element generator, to carry out the respective functions for which they are configured. The apparatus for generating the digital access element associated with a given polystyrene composition may be coupled to an entity producing the polystyrene composition. The apparatus for generating the digital access element associated with a given polystyrene composition may be coupled to an entity generating the digital access element on behalf of the data owner, such as the polystyrene composition producer.

The data providing service may comprise computer-executable instructions for providing and/or processing data, such as polystyrene composition data, associated with the data owner for accessing and/or processing by a data consuming service. The data consuming service may comprise computer-executable instructions for accessing and/or processing data, such as polystyrene composition data, associated with a data owner, such as the polystyrene composition data owner.

In an embodiment, the polystyrene composition associated with the digital access element may be selected from the group consisting of extruded polystyrene and at least partially expanded polystryene. The extruded polystyrene (XPS) may be an extruded foam. The extruded polystyrene may be obtainable commercially as Styrodur^{®} from BASF SE. The extruded polystyrene (XPS) may be produced via continuous extrusion of thermoplastic polymers, such as polystyrene or copolymers thereof. The fundamental steps in the process for the production of, for example, extruded polystyrene foam (XPS) is disclosed in "Polystyrol, Kunststoffhandbuch 4" [Polystyrene, Plastics handbook 4] [H. Gausepohl and R. Gellert, Hanser-Verlag, Munich (1996)), chapter 13.2.3 (pp. 591-598). Materials based on extruded polystytrene with uniform properties may be obtained by homogenizing the melt, for example by using static or dynamic mixers upstream of the die, in the extruder. The extruded polystyrene foam (XPS) may have a density range of 28 to 45 kg/m³.

The at least partially expanded polystyrene (EPS) may be present in the form of beads. The at least partially expanded polystyrene (EPS) may be pre-expanded polystyrene or fully expanded polystyrene. The pre-expanded polystyrene may be further expanded, such as fully expanded, with air. The pre-expanded polystyrene may comprise of from 50 to 99 percent air by volume. The pre-expanded polystyrene may be previously expanded with an organic blowing agent, such as a hydrocarbon like pentane, isopentane, butane and combinations thereof. Alternatively, the pre-expanded polymer may be previously expanded with an inorganic blowing agent, such an air, carbon dioxide, nitrogen, argon, and combinations thereof. The pre-expanded polystyrene may be greater than of about 50 percent expanded. The pre-expanded polystyrene may be greater than of about 60 percent expanded. The pre-expanded polystyrene may be greater than of about 70 percent expanded.

The pre-expanded polystyrene may be derived from expanded polymers, including thermoplastic polymers. Examples of pre-expanded polymers include polystyrene (e.g. free-radical-polymerized glass-clear polystyrene (GPPS) or anionically polymerized polystyrene (APS)), styrene-based-copolymers (e.g., styrene-maleic anhydride copolymers, styrene-butadiene copolymers, styrene-α-methylstyrene copolymers, acrylonitrile-butadiene-styrene (ABS) copolymers, styrene-acrylonitrile (SAN) copolymers, styrene-methyl methacrylate copolymers, acrylonitrile-styrene-acrylate (ASA) copolymers, methyl methacrylate-acrylonitrile-butadiene-styrene (MABS) copolymers), and combinations thereof. In some examples, the pre-expanded polystyrene includes a mixture of polystyrene and polyvinyl chloride. Examples of suitable commercially available pre-expanded polymers include NEOPOR and STYROPOR, expandable polystyrenes commercially available from BASF SE (Ludwigshafen am Rhein, Germany).

In an embodiment, the apparatus for generating the digital access element associated with the polystyrene composition may further comprise, i.e. apart from the digital identifier providing unit and the digital access element generator, a physical identifier providing unit configured to provide a physical identifier for the polystyrene composition, wherein the digital identifier is associated with the physical identifier. The physical identifier may refer, for instance, to a batch number and/or a LOT number of the polystyrene composition.

In an embodiment, the digital identifier is associated with the physical entity of the polystyrene composition. In this context the physical entity may relate to a polystyrene composition that is associated with the digital identifier. The digital identifier may be associated with the polystyrene composition via the physical identifier. The digital identifier may be associated with the polystyrene composition the polystyrene composition data is associated with. For instance, the digital identifier may be associated with the physical entity of the polystyrene composition.

In an embodiment, the apparatus for generating the digital access element associated with a respective polystyrene composition comprises an assigning unit, i.e. an assignor. This allows to associate the digital identifier to the physical identifier associated with the polystyrene composition. The assigning unit may be contained within the physical identifier providing unit or may be separate from said physical identifier providing unit. The assigning unit, or assignor, may be configured to assign a digital identifier to a physical identifier. Assigning may include generating a code having embedded the digital identifier received from the digital identifier providing unit. Assigning may include correlating the digital identifier received from the digital identifier providing unit with the physical identifier received from the physical identifier providing unit. For instance, an identifier element may be physically attached to a packaging of the polystyrene composition, wherein the identifier element may contain or correspond to the physical identifier. The identifier element may comprise a passive or active element such as, for instance, a barcode, a QR code an embossed code or an RFID tag. The physical identifier providing unit may be configured to provide the physical identifier based on the identifier element, such as by use of an ID reader.

In an embodiment, the digital identifier is associated with the physical entity the polystyrene composition will be supplied for and the polystyrene composition data is associated with. For instance, the digital identifier may be associated with the physical entity of the component, the component assembly, the end product or the like produced using the polystyrene composition. The digital identifier may be associated with more than one physical entity the polystyrene composition will be supplied for and the polystyrene composition data is associated with. For instance, the digital identifier may be associated with the physical entity of the component, the component assembly and the end product produced using the polystyrene composition. Associating the digital identifier with different physical entity stages in the chemical supply chain allows for virtually tracking the supplied polystyrene composition in the supply chain. This way polystyrene composition with its associated polystyrene composition data may be tracked e.g. up to the end of life of the end product.

In an embodiment, the apparatus may also comprise a collector configured to collect polystyrene composition data, wherein the digital identifier is associated with the collected polystyrene composition data. In an example, the collector may comprise manual, semiautomatic or automatic input means for inputting the polystyrene composition data. The input means may comprise one or more sensors, particularly measurement devices. The collector may be configured to collect the polystyrene composition data based on a physical identifier or a polystyrene composition identifier. If the physical identifier corresponds to an identifier element physically attached to a packaging of a polystyrene composition, for instance, the collector may comprise an ID reader for reading out the identifier element. More generally, the collector may comprise, or cooperate with, the physical identifier providing unit. For instance, the physical identifier providing unit may be configured to provide a physical identifier corresponding to an identifier element physically attached to packaging the polystyrene composition, wherein the collector may be configured to collect the polystyrene composition data based on the provided physical identifier. Optionally, the polystyrene composition data, or a part thereof, could be already encoded in the physical identifier, particularly in the identifier element physically attached to a packaging of the respective polystyrene composition, wherein the collector may be configured to collect the polystyrene composition data, or the part thereof, by decoding it from the identifier element.

In an embodiment, the digital identifier providing unit may be configured to provide the digital identifier upon receiving a request to provide at least said digital identifier. Hence, the digital identifier providing unit may provide, apart from the digital identifier, further data, such as access data, an owner or product identifier and/or authentication mechanism(s). The further data may be used by the digital access element generator to generate the digital access element, e.g. at least part of the further data may be included in the generated digital access element. The request may be received by the digital identifier unit via any communication channel, including but not limited to web servers, ad hoc WIFI, BLE beacon signals, NFC, a barcode, QR code RFID code scanning, etc. The request to provide the digital identifier may be associated with the polystyrene composition production producing the polystyrene composition. The request to provide the digital identifier may be generated by a computing system, such as an operating system, of polystyrene composition production producing the polystyrene composition. The request may be triggered at predefined occurrences. For instance, the packaging/filling line may comprise a detector detecting the physical identifier on each packaging unit of the polystyrene composition. Based on such recognition a computing apparatus, such as the operating system, of the polystyrene composition production may generate the request to provide the digital identifier. The generated request may be provided to the digital identifier providing unit.

The request to provide the digital identifier may include access data and/or an owner or product identifier associated with the polystyrene composition data owner or the polystyrene composition, respectively. The owner/product identifier may be a string identifier associated with a data owner name, such as the polystyrene composition producer, or the polystyrene composition name. The owner identifier, like the digital identifier associated with the polystyrene composition, may be provided based on a physical identifier, such as based on a barcode, a QR code, embossed code or a tag like an RFID tag. Through the owner identifier, the digital access element generated can be associated with the polystyrene composition data owner by including the owner identifier. Through the product identifier, the digital access element generated can be associated with the polystyrene composition by including the product identifier. The own-er/product identifier may be used for data transactions, such as sharing or exchanging polystyrene composition data. The owner/product identifier may be provided to a transaction manager. Providing the digital identifier and the owner/product identifier to a transaction manager or a data consuming service may simplify tracking of data transactions. Any transaction in the data ecosystem can e.g. be associated with the clear name of the data owner or the polystyrene composition.

The request to provide the digital identifier may further contain data associated with the detected physical identifier, such as the polystyrene composition ID or batch number. The request to provide the digital identifier may further contain an indication of the type of digital identifier, such as DID or UUID.

The request to provide the digital identifier may further contain an authentication mechanism, such as the public private key pair, and/or an identifier associated with the digital identifier providing unit and/or the digital access element generator. The authentication mechanisms may be retrieved from an authentication data storage, such as a vault, depending on the digital identifier to be requested.

In response to the request, the digital identifier providing unit may generate the digital identifier. Hence, the digital identifier providing unit may include a digital identifier generating unit. In response to the request, the digital identifier providing unit may request the digital identifier at a central node or one or more decentral nodes. The one central node or the decentral node(s) may - along with the apparatus described herein - be part of a decentral system comprising a plurality of member nodes. The generated digital identifier may further be provided to at least one authentication data registry node, preferably accessible by the data providing service and/or the data consuming service. The authentication data registry node may be a central registry node such as a central file system, a centrally managed distributed database, and/or a centrally managed peer-to-peer network. This enables customized data sharing or exchange with respect to the polystyrene composition and the chemical supply chain the polystyrene composition is supplied to. In particular, the data providing service and/or the data consuming service may customize data sharing or exchange protocols based on the anchoring of the digital identifier to the polystyrene composition data. The authentication data registry node may be a decentral registry such as a distributed ledger, a decentralized file system, a distributed database, and/or a peer-to-peer network. The decentral configuration allows for more efficient use of computing resources and strengthens control by the data owner. In addition, the decentral configuration is independent from centrally managed nodes and as such increases reliability and flexibility of the system. The authentication data registry node may be a central registry node such as a central file system, a centrally managed distributed database, and/or a centrally managed peer-to-peer network. The central configuration allows for more control and standardization via a central node.

In an embodiment, the digital access element may include the digital identifier and the access data. Hence, the digital access element generator may not only be configured to generate the digital access element based on the digital identifier provided by the digital identifier providing unit and access data, but also to generate the digital access element such that it includes the digital identifier and the access data. In this way, the generated digital access element associated with the respective polystyrene composition will include a) the digital identifier associated with the polystyrene composition and polystyrene composition data, and b) access data. This allows for a direct and therefore relatively simple transmittal of the digital identifier and the access data to a data consumer or any other party for which the digital access element may be generated. The generated digital access element may contain further data, such as an owner or product identifier. This allows to associate any transaction in the data ecosystem with the clear name of the data owner or the polystyrene composition. The generated access element may contain authentication mechanism(s) associated with the digital identifier.

In an embodiment, the access data allows for an access to at least part of the polystyrene composition data. Other parts of the polystyrene composition data may already be included in the digital access element. Such data may, however, only be accessible using the access data based on which the digital access element is generated. Other parts of the polystyrene composition data may not be accessible at all due to, for instance, not being acquired, collected, stored and/or digitalised. In one embodiment, the access data includes one or more digital representation(s) pointing to polystyrene composition data or parts thereof. In this context pointing means any network representation or address that is suitable for accessing the polystyrene composition data. The access data may include multiple digital representations pointing to distinct parts of the polystyrene composition data. The access data may include multiple digital representations pointing to different parts of the polystyrene composition data. Such different parts may overlap in some data points. The representation may include an access point to the polystyrene composition data, a link to access polystyrene composition data, an endpoint to access polystyrene composition data or a service endpoint to access polystyrene composition data. This way the polystyrene composition data can be maintained and controlled by a data owner, such as the polystyrene composition data owner. Access can be provided via the representation of an access point simplifying data verification, integrity checks or quality checks and access control, since not multiple distributed data points need to be checked and access controlled. The polystyrene composition data may be stored in a data base of or associated with the data owner. The polystyrene composition data may be stored in a data base accessible by the data owner. The digital representation pointing to polystyrene composition data or parts thereof may be associated with or relate to any such data base associated with or accessible by the data owner. For enhanced security the digital representation pointing to polystyrene composition data or parts thereof may indirectly relate to any such data base associated with or accessible by the data owner. Access data may include data relating to authentication and authorization schemes and/or cryptographic information such as, for instance, a public key for decrypting the polystyrene composition data. For instance, the access data may include any data used in authentication methods according to W3C recommendations, and/or any data needed for accessing data providing services like the ones known from IDS architectures, particularly their addresses.

In one embodiment, the digital access element is related to or includes one or more authentication mechanisms. The authentication mechanisms may be associated with the digital identifier and/or the access data. The authentication mechanism may directly or indirectly relate to the digital identifier and the access data. In an example of indirect relation, the authentication mechanism may relate to a certificate mechanism. For example, on access request by the data consuming service, a dynamic access token may be generated based on the certificate mechanism. Such dynamic access token may be used to open peer-to-peer communication channel between data consuming service and the data providing service. The authentication mechanism may include a token, such as private and public key infrastructure, a certificate mechanism or a biometric mechanism, such as fingerprints, face recognition or voice recognition or the like. One common public key certificate is for instance the X.509 certificate. Through the authentication mechanism data access by a data consuming service can be controlled in a secure manner and integrity of the data providing service can be ensured. This allows for more reliable, controlled and secure data exchange or sharing.

The one or more authentication mechanisms associated with the digital identifier may be provided to the digital access element generator and to at least one decentral authentication data registry, preferably accessible by the data providing service and/or the data consuming service. The authentication data registry may be a central registry such as a central file system, a centrally managed distributed database, and/or a centrally managed peer-to-peer network. The central configuration allows for higher control and standardization via a central node. The authentication data registry may be a decentral registry such as a distributed ledger, a decentralized file system, a distributed database, and/or a peer-to-peer network. The decentral configuration allows for more efficient use of computing resources and strengthens control by the data owner.

In one embodiment, the digital access element is related to or includes one or more authorization mechanisms. The authorization mechanism(s) may be associated with the decentral identifier and/or the access data. The authorization mechanisms may include authorization rule(s) including data transaction instructions or data transaction protocols, such as data usage policies, smart data contracts or mor complex data processing instructions associated with data providing and/or data consuming services. Through the authorization mechanism data access and data usage by a data consuming service can be controlled in a secure manner.

The one or more authorization mechanisms associated with the digital identifier may be provided to a node for generating or processing the digital access element or for accessing the polystyrene composition data. Additionally or alternatively, the one or more authorization mechanisms may be provided to at least one central or decentral authorization data registry, preferably accessible by the data providing service and/or the data consuming service. The one or more authorization mechanisms may be provided to a node generating or processing the digital access element and to at least one of a central file system, a centrally managed distributed database, a centrally managed peer-to-peer network, a distributed ledger, a decentralized file system, a distributed database, and/or a peer-to-peer network, preferably accessible by the data providing service and/or the data consuming service.

In an embodiment, the digital identifier providing unit is configured to provide - apart from the digital identifier associated with the polystyrene composition and the polystyrene composition data (hereinafter referred to as first digital identifier) - at least one precursor digital identifier associated with one or more precursor materials from which the polystyrene composition is produced. The digital identifier providing unit may be configured to generate a concatenation of the first digital identifier and precursor identifier(s) based on a relationship representation according to which the first digital identifier is associated with the precursor identifier(s). The relationship representation may be associated with a relationship between the polystyrene composition and each precursor material. The relationship representation may specify that the precursor material(s) may be used to produce the polystyrene composition and/or that the polystyrene composition may be produced by using the precursor material(s). One or more hash value(s) may be generated based on the relationship representation. Hash value(s) may be generated based on the concatenation of digital identifiers. Hash value(s) may be generated for the concatenation of digital identifiers. Hash value(s) may signify the concatenation of digital identifiers. The hash values may be generated based on data associated with the first digital identifier and the precursor material digital identifier(s). The hash value may be generated based on a combined data set associated with the first digital identifier and the precursor material digital identifiers. By concatenating digital identifiers associated with products of the chemical product supply chain and/or the recycling product chain, the physical entities can be tracked and traced virtually.

In an embodiment, the digital identifier providing unit is configured to provide the first digital identifier and the at least one precursor material identifier to the digital access element generator. The digital identifier providing unit may be configured to provide the first digital identifier and the at least one precursor material digital identifier to a retrieval data storage (also denoted as authentication registry within this disclosure). The request to provide the digital identifier may include the one or more precursor digital identifiers. The request to provide the digital identifier may include data associated with the physical identifier of the polystyrene composition. Based on said data associated with the physical identifier, the digital identifier providing unit may retrieve the one or more precursor digital identifiers.

A digital identifier associated with a precursor material may be associated with precursor material data indicative of whether the precursor material is a virgin or a recycled material, of an environmental impact and/or of an origin of the precursor material. The digital identifiers associated with the precursor materials may be determined based on physical identifiers possibly corresponding to physical identifying elements like tags attached to the packaging of the precursor materials, for instance. Hence, the digital identifier providing unit may be configured to determine the digital identifiers associated with the respective precursor materials.

The precursor materials may be raw materials, including virgin or recycled materials. Raw materials may include styrene, polymerization initiators, solvents, blowing agents, additives or the like. The precursor materials may be used directly for producing the polystyrene composition.

The precursor materials may be used indirectly for producing the polystyrene composition. That is to say, some of the precursor materials may be used for producing one or more further intermediate products based on which, in turn, the polystyrene composition for which the digital identifier is to be provided is produced as described previously. Hence, at least part of the precursor materials used to produce the polystyrene composition may be intermediate products manufactured from raw material(s) and/or other intermediate product(s). Intermediate products may include polystyrene polymers and copolymers thereof and/or pre-expanded polystyrene. The polystyrene composition may be produced from raw materials and intermediate products. For instance, the extruded polystyrene may be produced from polystyrene polymer(s) and/or copolymer(s) thereof and additives. In another instance, the at least partially expanded polystyrene may be produced from polystyrene polymer(s) and/or copolymer(s) thereof and blowing agent(s).

In an embodiment, the digital access element generator may be configured to generate the digital access element based on the first digital identifier (e.g. the digital identifier associated with the polystyrene composition and the polystyrene composition data) and the at least one precursor identifier or the generated concatenation. This allows to link the digital identifier associated with the polystyrene composition with precursor digital identifiers associated with precursor materials used to produce the polystyrene composition. Hence, the relationship between the polystyrene composition and precursor materials used to produce the polystyrene composition can be reflected within the digital access element.

In an embodiment, the polystyrene composition data includes data on any of: the one or more precursor materials and the polystyrene composition itself. The expression "any of", when followed by a list of elements, is to be understood herein to include any one of the listed elements individually as well as any combination of the listed elements.

The data on the one or more precursor materials may include data indicative of any of the following:
- data on the transport of the precursor materials to the polystyrene composition manufacturing location
- origin of precursor materials
- physical identifier of the precursor material, such as charges/batches,
- chemical composition of the precursor material and/or its components,
- certifying documents/information,
- emission data of the precursor material,
- recycled content data of the precursor material,
- production data, such as data related to the production of the precursor material.
Production of precursor materials may include recycling processes. Recycling processes may include re-use processes and recycling processes. Recycling processes may include a physical and/or chemical treatment of the material to be recycled. For instance, styrene contained in used polystyrene compositions may be recovered by chemical processes. Production data may include monitoring and/or control data associated with the production of the precursor material. Production data may include measurement data related to a quality of the precursor material. Production data may include time and location data associated with the production

The data on the polystyrene composition for which the digital access element is generated may include data indicative of any of the following:
- batch number
- production data, such as production data previously described
- origin of raw materials or precursor materials
- product specifications,
- polystyrene composition technical data, such as technical data sheet (TDS),
- certificate data, such as fire certificate data, halal certificate data
- physical data, such as melting point, boiling point, density, hardness, flammability, shelf life, pH value, thermal conductivity mean values,
- polystyrene composition safety data, such as material safety data sheet (MSDS),
- emission data
- recycled content data
- packaging data, such as size
- data on the transport and handling of the polystyrene composition, such as recycling instructions, unloading instructions

In one embodiment, the digital access element includes data related to different class(es) of polystyrene composition data. For instance, the access data may include multiple digital representations pointing to different classes of the polystyrene composition data. For instance, at least one class of data on the polystyrene composition itself may include data required by regulation or regulatory data for chemicals, such as polystyrene composition technical data, polystyrene composition safety data and fire certificate data. At least one further class of data on the polystyrene composition itself may include emission data, recycled content data, halal certificate data and/or thermal conductivity mean values. Access to the data on the polystyrene composition itself may be controlled based on said classes. For instance, access to emission data, recycled content data, halal certificate data, thermal conductivity mean values or combinations thereof may be restricted while access to data required by regulation or regulatory data may not be restricted. Such access restriction may be provided by an authorization mechanism or scheme. For instance, the authorization mechanism or scheme may include a rule that specifies which data consuming services get access under which conditions.

In an embodiment, access to the polystyrene composition data via the digital access element may be controlled by a data providing service. The data providing service may be associated with the data owner of the polystyrene composition data, such as the polystyrene composition producer. This allows to retain full control over the polystyrene composition data by the data owner but at the same time enabling sharing of the polystyrene composition data under controlled conditions, for example by using appropriate authorization and authentication mechanisms or schemes, to improve production processes, such as discrete manufacturing, or recycling processes of discrete products containing the polystyrene composition associated with the polystyrene composition data.

In an embodiment, the generated digital access element may be provided for access by a data consuming service. The data consuming service may be owned or controlled by a consumer of the polystyrene composition, such as a discrete product producer or a participant of the recycling process of the discrete product. Discrete products may be broken down at the end of its lifecycle so that its components can be recycled. Examples of discrete products include machines, shoes, etc. The polystyrene composition data received by the data consuming service may be stored on a dedicated storage owned or controlled by the entity owning or controlling the data consuming service. Via the data consuming service, the discrete product processor may be able to retrieve the polystyrene composition data associated with received polystyrene composition(s). Use of the polystyrene composition data associated with the digital access elements) during production of discrete materials or components thereof may allow to improve production processes using said data, for example by controlling the production processes based on said polystyrene composition data, to enhance the properties of the resulting component or discrete product or the overall production efficiency. Use of the polystyrene composition data associated with the digital access element(s) during recycling processes allows to reliably determine the chemical composition of the polystyrene composition to be recycled, thus improving recycling efficiency by determining the correct recycling process, recycling parameters, recycling plant, etc.

In an embodiment, the apparatus for generating the digital access element is comprised in an apparatus for producing the polystyrene composition, i.e. in a production apparatus. The production apparatus may refer to one or more reactors or other equipment in a production facility producing the polystyrene composition. The production apparatus may refer to a production network comprising multiple production chains. During production of the polystyrene composition, or in the course of its subsequent packaging, a physical identifier may be attached to it, either manually, semi-automatically or automatically. The digital identifier providing unit may be configured to provide the digital identifier for the produced polystyrene composition based on this physical identifier as previously described. Moreover, polystyrene composition data may be collected before, during and/or after production of the polystyrene composition, such as using a collector as indicated above. The provided digital identifier may hence be associated with this polystyrene composition data. Thus, apart from the polystyrene composition being physically produced, a digital access element is generated for the produced polystyrene composition.

The apparatus for generating the digital access element, and thereby also the apparatus for producing the polystyrene composition and comprising the apparatus for generating the digital access element, may cooperate with an apparatus for managing digital access elements for polystyrene composition, particularly for managing those digital access elements which have been generated by the apparatus for generating digital access elements. Instead of cooperating with a further apparatus, a single combined apparatus may be provided, wherein the single combined apparatus may fulfil the functions of generating and managing the digital access elements. The apparatus for managing digital access elements, or the single combined apparatus, may comprise a digital access elements storage for storing the digital access elements and/or a retrieval data storage for storing retrieval data associated with the digital access elements. The retrieval data may allow for a retrieval of the digital access elements from the digital access elements storage. The retrieval data may allow for retrieval of the polystyrene composition data associated with generated digital access elements from respective data providing service using the retrieval data by a data consuming service. By storing the digital access elements and allowing for a retrieval of the stored digital access elements, a necessity to repeatedly generate the digital access elements may be avoided, which can simplify exchanging and sharing of polystyrene composition data.

It may also be preferable to not store the digital access elements. This may increase data safety. Then, the apparatus for managing the digital access elements, or the single combined apparatus, would not comprise the digital access element storage for storing the digital access elements. Moreover, the retrieval data storage would store retrieval data associated with the digital access elements, wherein now the retrieval data would allow not, i.e. not directly, for a retrieval of the digital access elements from a digital access element storage, but for generating digital access element based thereon, particularly for recovering digital identifiers, access data and/or polystyrene composition data. The data stored on the retrieval data storage may be stored according to digital identifiers associated with the data, wherein the digital identifiers indicate respective polystyrene composition s to which the respective stored retrieval data relate. Hence, retrieval data relating to a polystyrene composition with a given digital identifier may be retrieved from the retrieval data storage based on the digital identifier.

In an embodiment, the stored retrieval data comprises the digital identifier(s) and associated access data. For instance, based on the decentral identifier, the associated access data may be retrieved. The stored retrieval data may comprise representations of digital identifiers, particularly hash values determined based on digital identifiers. The stored retrieval data may comprise digital identifier(s) and associated data related to the polystyrene composition, such as a physical identifier. For instance, based on the physical identifier, the associated decentral identifier may be retrieved. The retrieval data may comprise a concatenation generated from the digital identifier associated with the polystyrene composition and the precursor digital identifier(s) based on a relationship representation as previously described. For instance, the concatenation may be represented by a hash value.

In an embodiment, the retrieval data storage corresponds to a distributed ledger or a database. Since the retrieval data may contain authentication information associated with digital access elements and/or digital identifiers contained in digital access elements, the retrieval data store may be regarded as an authentication registry. The retrieval data store may be configured to resolve the concatenation contained in the retrieval data. For instance, the retrieval data store may be configured to resolve, based on the relationship representation, the concatenation and to provide data on the precursor materials used to produce the polystyrene composition. Said data may include the precursor material digital identifiers. Hence, the retrieval data store may allow to determine the precursor materials used to produce the polystyrene composition. Moreover, the retrieval data store may allow to determine the materials used to produce a component, a component assembly or an end product, facilitating track and trace of materials along the entire value chain.

### BRIEF DESCRIPTION OF THE DRAWINGS

In the following, the present disclosure is further described with reference to the enclosed figures:
- FIGs. 1a,b: illustrate example embodiments of a centralized and a decentralized computing environment with computing nodes.
- FIG. 1c: illustrate an example embodiment of a distributed computing environment.
- FIGs. 2a,2b: illustrate an example of a polystyrene composition production controlled by an operating system containing an apparatus for generating a digital access element associated with a polystyrene composition.
- FIG. 3: shows schematically and exemplarily an apparatus and associated methods for generating a digital access element in connection with a polystyrene composition produced by a chemical production network.
- FIG. 4: shows schematically and exemplarily a relation of a digital access element to further data structures.
- FIG. 5: shows schematically and exemplarily a further relation of a digital access element to further data structures.
- FIG. 6: shows an example method for generation of the digital access element.
- FIG. 7: shows an example method for using the digital access element to further process the polystyrene composition associated with the digital access element.
- FIG. 8: shows an example method for authorizing access to polystyrene composition data.
- FIG. 9: shows a schematic illustration of a method or apparatus for providing polystyrene composition data associated with polystyrene composition(s) to a polystyrene composition consumer as data consumer via a decentral network.
- FIG. 10: schematically an example of a method or apparatus for providing polystyrene composition data associated with a polystyrene composition to value chains participants via the decentral network
- FIGs. 11-16: show schematically and exemplarily possible relations between digital access elements.
- FIG. 17A,17B: illustrate examples of relationship representations, which may be used for generating a concatenation.
- Fig. 18: illustrates an example of an apparatus for generating and using a concatenation in a decentral network.

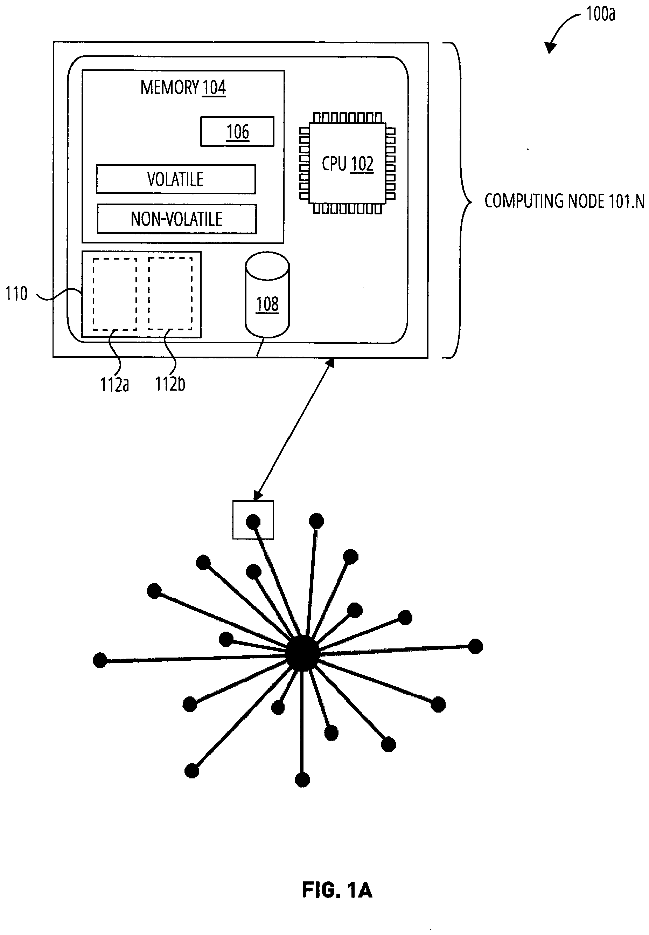

FIG. 1a to FIG. 1c illustrate different computing environments, central, decentral and distributed. The methods, apparatuses, systems, digital access elements and computer elements of this disclosure may be implemented in decentral or at least partially decentral computing environments. In particular, for data sharing or exchange in ecosystems of multiple players different challenges exist. Data sovereignty may be viewed as a core challenge. It can be defined as a natural person's or corporate entity's capability of being entirely self-determined with regard to its data. To enable this particular capability related aspects, including requirements for secure and trusted data exchange in business ecosystems, may be implemented across the chemical value chain. In particular, chemical industry requires tailored solutions to deliver chemical products, such as polystyrene composition s, in a more sustainable way by using digital ecosystems.

FIG. 1a illustrates an example embodiment of a centralized computing system 100a comprising a central computing node (filled circle in the middle) and several peripheral computing nodes 101.1 to 101.N (denoted as filled circles in the periphery). The computing system may include one or more computing nodes, a system of nodes or combinations thereof. Computing node may refer to any device or system that includes at least one physical and tangible processor, and a physical and tangible memory capable of having thereon computer-executable instructions that are executed by a processor. Computing nodes are now increasingly taking a wide variety of forms. Computing nodes may, for example, be handheld devices, production facilities, sensors, monitoring systems, control systems, appliances, laptop computers, desktop computers, mainframes, data centers, or even devices that have not conventionally been considered a computing node, such as wearables (e.g., glasses, watches or the like). The memory may take any form and may depend on the nature and form of the computing node.

In this example, the peripheral computing nodes 101.1 to 101.N may be connected to one central computing system (or server). In another example, the peripheral computing nodes 101.1 to 1 01.N may be attached to the central computing node via e.g. a terminal server (not shown). The majority of functions may be carried out by or obtained from the central computing node (also called remote centralized location). One peripheral computing node 101.N has been expanded to provide an overview of the components present in the peripheral computing node 101.N. The central computing node may comprise the same components as described in relation to the peripheral computing node 101.N.

Each computing node 101, 101.1 to 101. N may include at least one hardware processor 102 and memory 104. Processor may refer to an arbitrary logic circuitry configured to perform basic operations of a computer or system, and/or, generally, to a device which is configured for performing calculations or logic operations. In particular, the processor, or computer processor may be configured for processing basic instructions that drive the computer or system. It may be a semiconductor based processor, a quantum processor, or any other type of processor configured for processing instructions. As an example, the processor may comprise at least one arithmetic logic unit ("ALU"), at least one floating-point unit ("FPU)", such as a math coprocessor or a numeric coprocessor, a plurality of registers, specifically registers configured for supplying operands to the ALU and storing results of operations, and a memory, such as an L1 and L2 cache memory. In particular, the processor may be a multicore processor. Specifically, the processor may be or may comprise a Central Processing Unit ("CPU"). The processor may be a ("GPU") graphics processing unit, ("TPU") tensor processing unit, ("CISC") Complex Instruction Set Computing microprocessor, Reduced Instruction Set Computing ("RISC") microprocessor, Very Long Instruction Word ("VLIW") microprocessor, or a processor implementing other instruction sets or processors implementing a combination of instruction sets. The processing means may also be one or more special-purpose processing devices such as an Application-Specific integrated Circuit ("ASIC"), a Field Programmable Gate Array ("FPGA"), a Complex Programmable Logic Device ("CPLD"), a Digital Signal Processor ("DSP"), a network processor, or the like. The methods, apparatuses, systems and devices described herein may be implemented as software in a DSP, in a micro-controller, or in any other side-processor or as hardware circuit within an ASIC, CPLD, or FPGA. It is to be understood that the term processor may also refer to one or more processing devices, such as a distributed system of processing devices located across multiple computer systems (e.g., cloud computing), and is not limited to a single device unless otherwise specified.

The memory 104 may refer to a physical system memory, which may be volatile, non-volatile, or a combination thereof. The memory may include non-volatile mass storage such as physical storage media. The memory may be a computer-readable storage media such as RAM, ROM, EEPROM, CD-ROM, or other optical disk storage, magnetic disk storage, or other magnetic storage devices, or any other physical and tangible storage medium which can be used to store desired program code means in the form of computer-executable instructions or data structures and which can be accessed by the computing system. Moreover, the memory may be a computer-readable media that carries computer- executable instructions (also called transmission media). Further, upon reaching various computing system components, program code means in the form of computer-executable instructions or data structures can be transferred automatically from transmission media to storage media (or vice versa). For example, computer-executable instructions or data structures received over a network or data link can be buffered in RAM within a network interface module (e.g., a "NIC"), and then eventually transferred to computing system RAM and/or to less volatile storage media at a computing system. Thus, it should be understood that storage media can be included in computing components that also (or even primarily) utilize transmission media.

The computing nodes 101, 101.1...101.N may include multiple structures 106 often referred to as an "executable component or computer-executable instructions". For instance, memory 104 of the computing nodes 101, 101.1...101.N may be illustrated as including executable component 106. The term "executable component" may be the name for a structure that is well understood to one of ordinary skill in the art in the field of computing as being a structure that can be software, hardware, or a combination thereof or which can be implemented in software, hardware, or a combination. For instance, when implemented in software, one of ordinary skill in the art would understand that the structure of an executable component includes software objects, routines, methods, and so forth, that is executed on the computing nodes 101, 101.1...101. N, whether such an executable component exists in the heap of a computing node 101, 101.1...101.N, or whether the executable component exists on computer-readable storage media. In such a case, one of ordinary skill in the art will recognize that the structure of the executable component exists on a computer-readable medium such that, when interpreted by one or more processors of a computing node 101, 101.1...101.N (e.g., by a processor thread), the computing node 101, 101.1...101.N is caused to perform a function. Such a structure may be computer-readable directly by the processors (as is the case if the executable component were binary). Alternatively, the structure may be structured to be interpretable and/or compiled (whether in a single stage or in multiple stages) to generate such binary that is directly interpretable by the processors. Such an understanding of example structures of an executable component is well within the understanding of one of ordinary skill in the art of computing when using the term "executable component". Examples of executable components implemented in hardware include hardcoded or hard-wired logic gates, that are implemented exclusively or near-exclusively in hardware, such as within a field- programmable gate array (FPGA), an application-specific integrated circuit (ASIC), or any other specialized circuit. In this description, the terms "component", "agent", "manager", "service", "engine", "module", "virtual machine" or the like are used synonymous with the term "executable component.

The processor 102 of each computing node 101, 101.1...101.N may direct the operation of each computing node 101, 101.1...101.N in response to having executed computer- executable instructions that constitute an executable component. For example, such computer-executable instructions may be embodied on one or more computer-readable media that form a computer program product. The computer-executable instructions may be stored in the memory 104 of each computing node 101, 101.1...101.N. Computer-executable instructions comprise, for example, instructions and data which, when executed at a processor 101, cause a general purpose computing node 101, 101.1...101.N, special purpose computing node 101, 101.1...101.N, or special purpose processing device to perform a certain function or group of functions. Alternatively, or in addition, the computer-executable instructions may configure the computing node 101, 101.1...101.N to perform a certain function or group of functions. The computer executable instructions may be, for example, binaries or even instructions that undergo some translation (such as compilation) before direct execution by the processors, such as intermediate format instructions such as assembly language, or even source code.

Each computing node 101, 101.1...101.N may contain communication channels 108 that allow each computing node 101.1... 101.N to communicate with the central computing node, for example, a network (depicted as solid line between peripheral computing nodes and the central computing node in Fig. 1a). A network may be defined as one or more data links that enable the transport of electronic data between computing nodes 101, 101.1...101.N and/or modules and/or other electronic devices. When information is transferred or provided over the network or another communications connection (either hardwired, wireless, or a combination of hardwired or wireless) to a computing node 101, 101.1...101.N, the computing node 101, 101.1...101.N may view the connection as a transmission medium. Transmission media can include the network and/or data links which can be used to carry desired program code means in the form of computer-executable instructions or data structures and which can be accessed by a general-purpose or special-purpose computing nodes 101, 101.1...101.N. Combinations of the above may also be included within the scope of computer-readable media.

The computing node(s) 101, 101.1 to 101.N may further comprise a user interface system 110 for use in interfacing with a user. The user interface system 110 may include output mechanisms 110a as well as input mechanisms 110b. The principles described herein are not limited to the precise output mechanisms 110a or input mechanisms 110b as such will depend on the nature of the device. However, output mechanisms 110a might include, for instance, displays, speakers, displays, tactile output, holograms and so forth. Examples of input mechanisms 110b might include, for instance, microphones, touchscreens, holograms, cameras, keyboards, mouse or other pointer input, sensors of any type, and so forth.

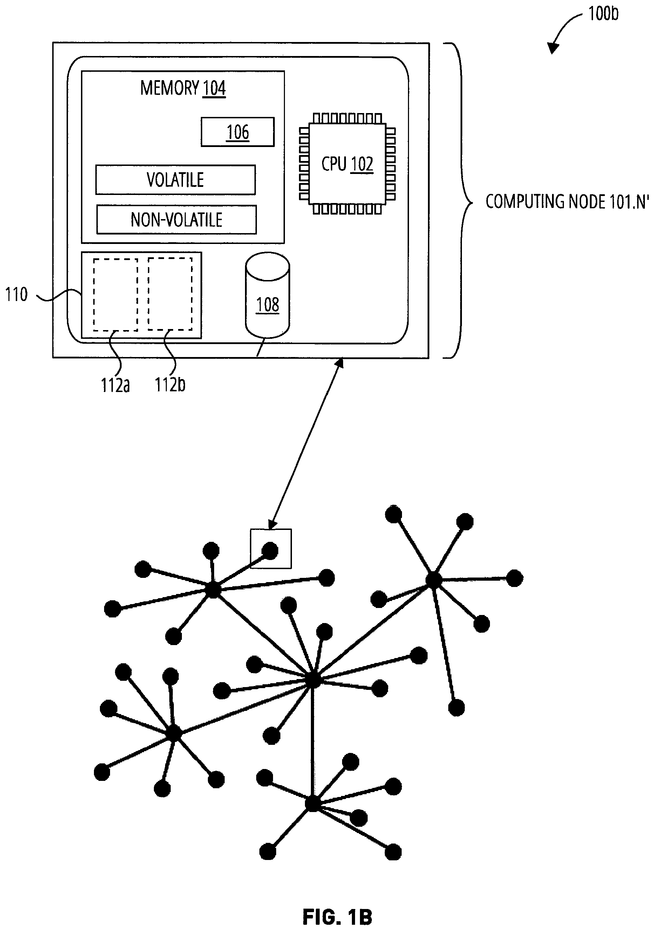

FIG. 1b illustrates an example embodiment of a decentralized computing environment 100b with several computing nodes 101.1' to 101.N' denoted as filled circles. In contrast to the centralized computing environment 100a illustrated in FIG. 1a, the computing nodes 101.1' to 101.N' of the decentralized computing environment are not connected to a central computing node and are thus not under control of a central computing node. Instead, resources, both hardware and software, may be allocated to each individual computing node 101.1'...101.N' (local or remote computing system) and data may be distributed among various computing nodes 101.1'...101.N' to perform the tasks. Thus, in a decentral system environment, program modules may be located in both local and remote memory storage devices. One computing node 101.N' has been expanded to provide an overview of the components present in the computing node 101.N'. In this example, the computing node 101.N' comprises the same components as described in relation to FIG. 1a.

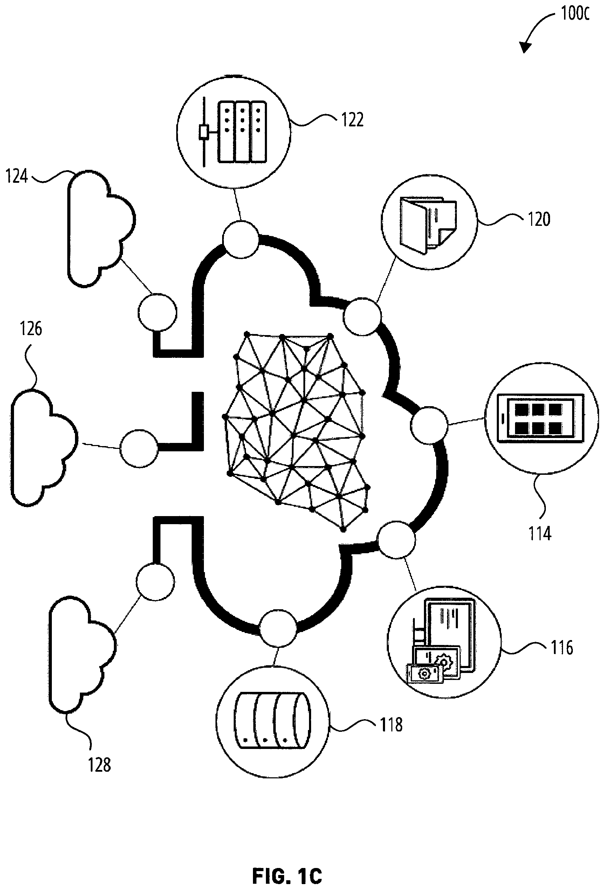

FIG. 1c illustrates an example embodiment of a distributed computing environment 100c. In this description, "distributed computing" may refer to any computing that utilizes multiple computing resources. Such use may be realized through virtualization of physical computing resources. One example of distributed computing is cloud computing. Cloud computing may refer a model for enabling on-demand network access to a shared pool of configurable computing resources (e.g., networks, servers, storage, applications, and services). When distributed, cloud computing environments may be distributed internationally within an organization and/or across multiple organizations. In this example, the distributed cloud computing environment 100c may contain the following computing resources: mobile device(s) 114, applications 116, databases 118, data storage 120 and server(s) 122. The cloud computing environment 100c may be deployed as public cloud 124, private cloud 126 or hybrid cloud 128. A private cloud 124 may be owned by an organization and only the members of the organization with proper access can use the private cloud 126, rendering the data in the private cloud at least confidential. In contrast, data stored in a public cloud 126 may be open to anyone over the internet. The hybrid cloud 128 may be a combination of both private and public clouds 124, 126 and may allow to keep some of the data confidential while other data may be publicly available.

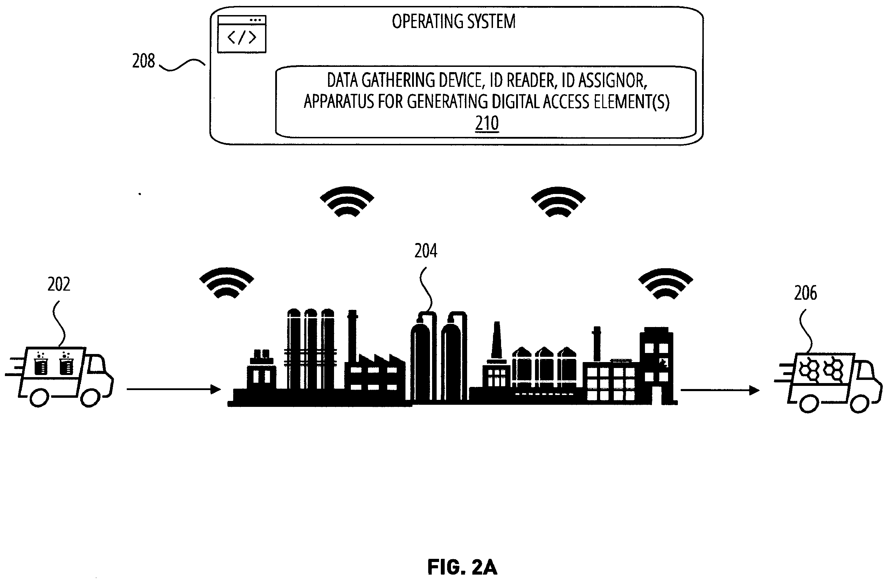

FIG. 2A illustrates an example of a polystyrene composition production 204 controlled by an operating system 208 containing an apparatus for generating a digital access element associated with a polystyrene composition. The operating system 208 may be used to operate the polystyrene composition production 204, for example by managing different production chains present within the polystyrene composition production. The polystyrene composition production 204 may produce polystyrene composition(s) from one or more precursor materials, for example by reacting one or more of the precursor materials and/or by physically processing one or more precursor materials. The precursor materials may be inbound materials 202 or may be intermediate products produced from said inbound materials 202 by the polystyrene composition production 204. The polystyrene composition may be extruded polystyrene (XPS). The polystyrene composition may be at least partially expanded polystyrene (EPS), such as pre-expanded polystyrene or fully expanded polystyrene.

For producing one or more polystyrene composition(s) 206, different inbound materials 202 hereinafter may be provided as physical inputs from material providers or suppliers. The physical inputs to the polystyrene composition production 204 may include precursor materials, such raw materials, intermediate materials or a combination thereof. Raw materials may be virgin or recycled raw materials.

The polystyrene composition production 204 may convert inbound material 202 by way of chemical conversion and/or by way of physical processing to one or more polystyrene composition(s) 206, that exit the polystyrene composition production 204. The chemical conversion may be performed via intermediate products or components. The conversion may be a chemical reaction or any other processing step.

The polystyrene composition production 204 may include multiple production steps. The production steps included in the polystyrene composition production 204 may be defined by the system boundary of the polystyrene composition production 204. The system boundary may be defined by location or control over production processes. The system boundary may be defined by the site of the polystyrene composition production 204. The system boundary may be defined by production processes controlled by one entity or multiple entities jointly. The system boundary may be defined by value chain with staggered production processes to the polystyrene composition, which may be controlled by multiple entities separately.

The operating system 208 of the polystyrene composition production 204 may monitor and/or control the polystyrene composition production 204 based on operating parameters associated with the different processes performed by the polystyrene composition production 204. One process step monitored and/or controlled may be the feed of inbound materials and/or the release of produced polystyrene composition(s). Another process step monitored and/or controlled may the generation of digital access elements. Yet another process step monitored and/or controlled may be the provisioning of the generated digital access elements for access by a data consuming service.

The operating system 208 may be configured to collect polystyrene composition data before, during and/or after production of polystyrene composition by polystyrene composition production 204. The polystyrene composition data may include data on the precursor materials, such as inbound material(s) 202 and intermediate product(s), and/or the produced polystyrene composition. For instance, the polystyrene composition data could be indicative of data relating to precursor materials 202 used in the production of the polystyrene composition 206, including transport data of the precursor materials 202. For collecting the polystyrene composition data, the operating system 208 may comprise a data gathering device which is configured to collect the polystyrene composition data. The data gathering device may, for instance, wirelessly receive production and/or transport data from data acquisition devices at a plurality of locations in and around the polystyrene composition production 204. The gathered polystyrene composition data may be interrelated with a polystyrene composition identifier and may be stored in a database. The database may be associated with the polystyrene composition production 204 and/or operating system 208.

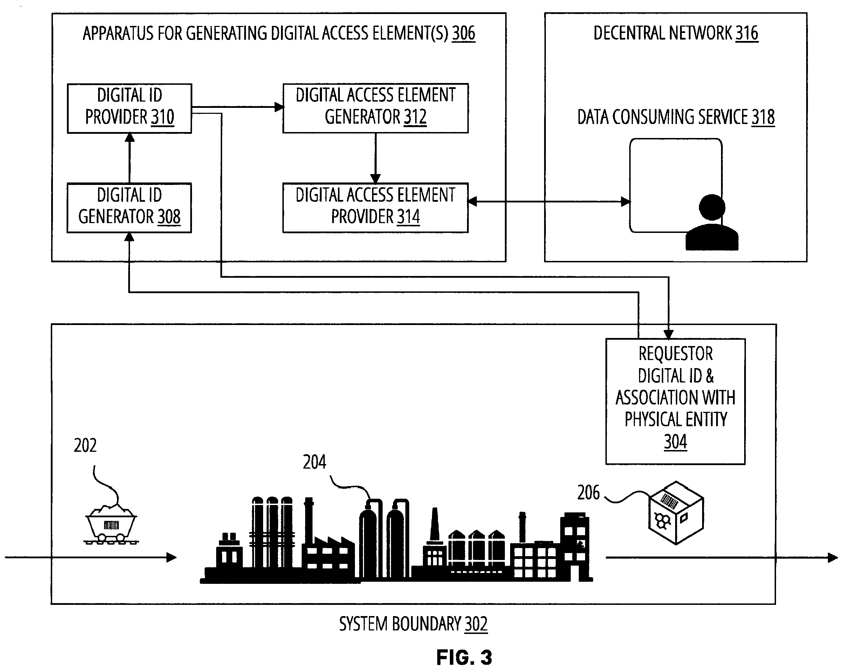

The operating system 208 may be configured to generate the digital access element using the apparatus for generating digital access element(s). While not explicitly indicated in FIG. 2A, the apparatus may comprise a digital identifier providing unit configured to provide a digital identifier associated with the polystyrene composition 206 and polystyrene composition data, such as polystyrene composition data collected by the data gathering device, and a digital access element generator configured to generate the digital access element based on the digital identifier and access data. For instance, the apparatus may be configured to generate the digital access element upon receiving a request to provide at least the digital identifier as described in relation to FIG. 3 below.

While not being shown in FIG. 1, physical identifier elements have been attached to each packaging of the polystyrene composition. Said physical identifier elements correspond to physical identifiers of the polystyrene composition, such as physical identifiers for the batch of produced polystyrene composition 206. The operating system 208 may be configured to determine the physical identifier associated with produced polystyrene composition(s) 206. The operating system 208 may comprise an ID reader configured to read the physical identifier element physically connected to the produced polystyrene composition. For instance, the physical identifier may be provided by scanning RFID tags serving as physical identifier elements attached to the packaging of the polystyrene composition.

The digital identifier providing unit may be configured to provide the digital identifier. The digital identifier providing unit be configured to provide - apart from the digital identifier associated with the polystyrene composition and the polystyrene composition data (hereinafter referred to as first digital identifier) - at least one precursor digital identifier associated with one or more precursor materials from which the polystyrene composition is produced. The digital identifier providing unit may be configured to generate a concatenation of the first digital identifier and precursor identifier(s) based on a relationship representation according to which the first digital identifier is associated with the precursor identifier(s). The relationship representation may be associated with a relationship between the polystyrene composition and each precursor material. The relationship representation may specify that the precursor material may be used to produce the polystyrene composition and/or the polystyrene composition may be produced by using the precursor material. Hence, how the digital identifiers of the polystyrene composition and the precursor materials are concatenated may depend, for instance, on a relation between the polystyrene composition and the precursor materials in the supply chain and/or to how the polystyrene composition is produced from the precursor materials. Moreover, if the precursor materials relate to several stages in the supply chain, such that some of the precursor materials are themselves used in the production of other precursor materials, concatenation may be performed for one or more stages. For instance, each of the precursor digital identifiers may be concatenated with the digital identifier of the polystyrene composition. Alternatively, each of the precursor digital identifiers may be concatenated with the digital identifier associated with the intermediate product produced from precursor materials associated with said digital identifiers, and the resulting concatenation may be concatenated with the digital identifier of the polystyrene composition. One or more hash value(s) may be generated based on the relationship representation. Hash value(s) may be generated based on the concatenation of digital identifiers. Hash value(s) may be generated for the concatenation of digital identifiers. Hash value(s) may signify the concatenation of digital identifiers. The hash values may be generated based on data associated with a first digital identifier and the at least one precursor digital identifier. The hash value may be generated based on a combined data set associated with the first digital identifier and the precursor material digital identifiers. By concatenating digital identifiers associated with products of the chemical product supply chain and/or the recycling product chain, the physical entities can be tracked and traced virtually.

The access data may refer to any data needed for accessing the polystyrene composition data. Hence, the type of access data can vary significantly between embodiments. Examples of access data include digital representation(s) pointing to the polystyrene composition data or parts thereof. Such digital representation(s) may include endpoint addresses of data providing services associated with the polystyrene composition data, e.g. having access to data structures storing the respective polystyrene composition data or parts thereof. Access data may include data needed in or indicative of an authentication mechanism and/or an authorization mechanism to be carried out before being able to access the polystyrene composition data.

The operating system 208 may be configured to assign the provided digital identifier to the physical identifier associated with the produced polystyrene composition 206. The operating system 208 may comprise an ID assignor configured to assign the provided digital identifier to the physical identifier associated with the polystyrene composition, such as the batch of produced polystyrene composition 206. Assigning may include generating a code having embedded the digital identifier received from the digital identifier providing unit. Assigning may include correlating the digital identifier received from the digital identifier providing unit with the physical identifier received from the physical identifier providing unit. Assigning may result in association of the digital identifier to the polystyrene composition data since the polystyrene composition data is associated with the polystyrene composition.

The data gathering device, the ID assignor, the ID reader and the apparatus for generating digital access element(s) associated with polystyrene composition(s) may be configured as decentral services or applications executed via the decentral network.

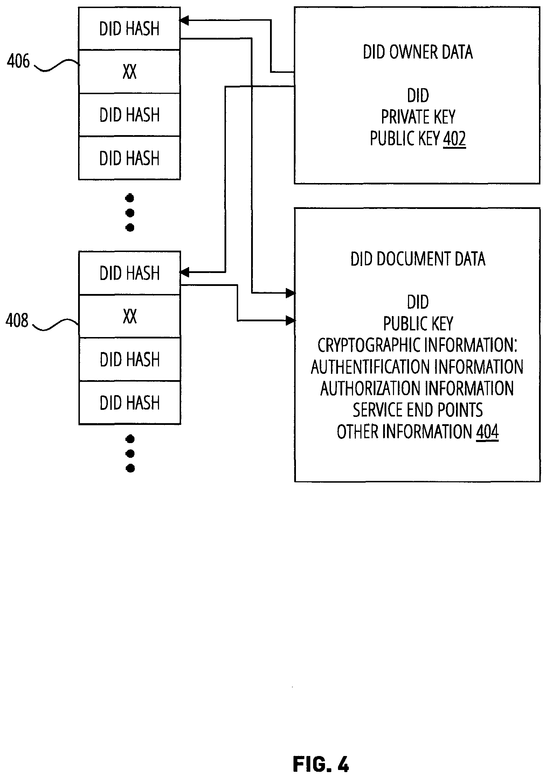

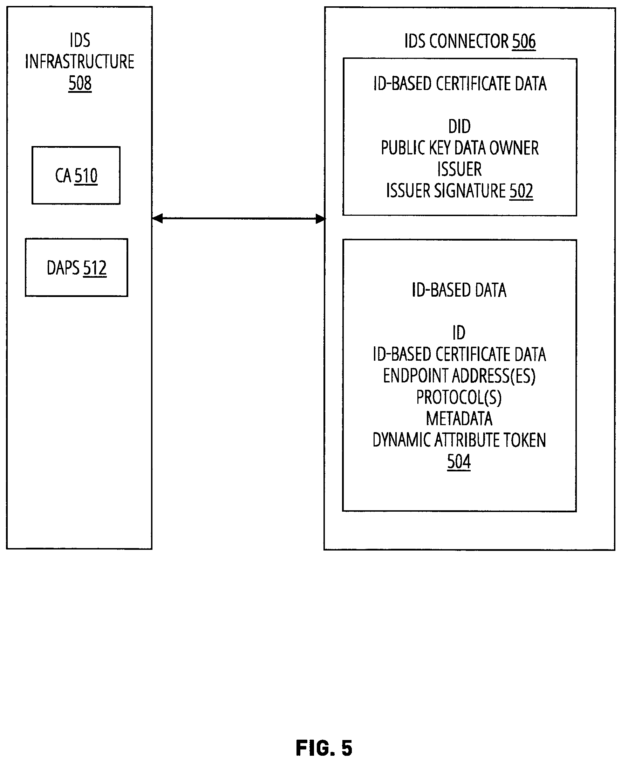

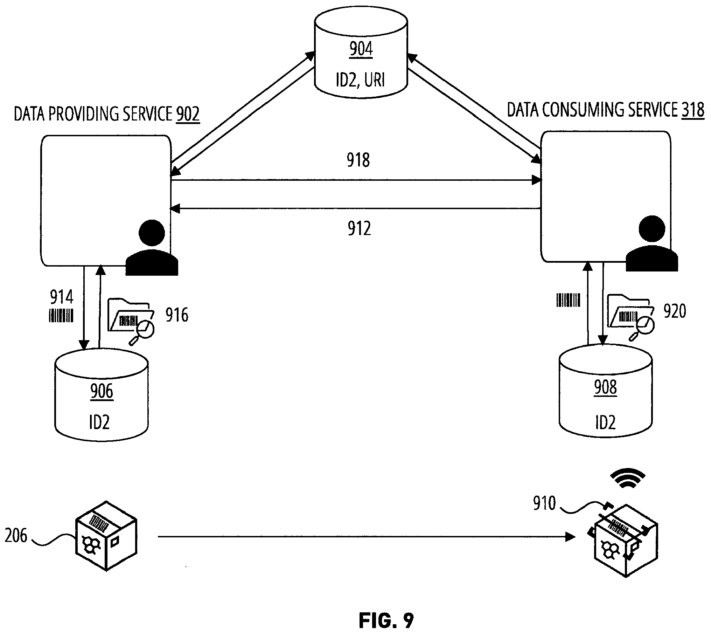

The digital access element generated by the apparatus may be a digital document or other digital data structure including the digital identifier and the access data, such as digital access elements shown in FIG. 4 and FIG. 5. It should be emphasized already, however, that the digital access element may also include a part of the polystyrene composition data itself. Hence, some of the polystyrene composition data may be included in the generated digital access element and thereby directly provided to a data consumer, such as a polystyrene composition recipient, while other of the polystyrene composition data, which may be more sensitive and therefore more security relevant data, may not be included in the generated digital access element, but only be accessible based on the digital access element upon conditions to be met by, for instance, the data consuming service associated with the data consumer (see for example FIG. 9).

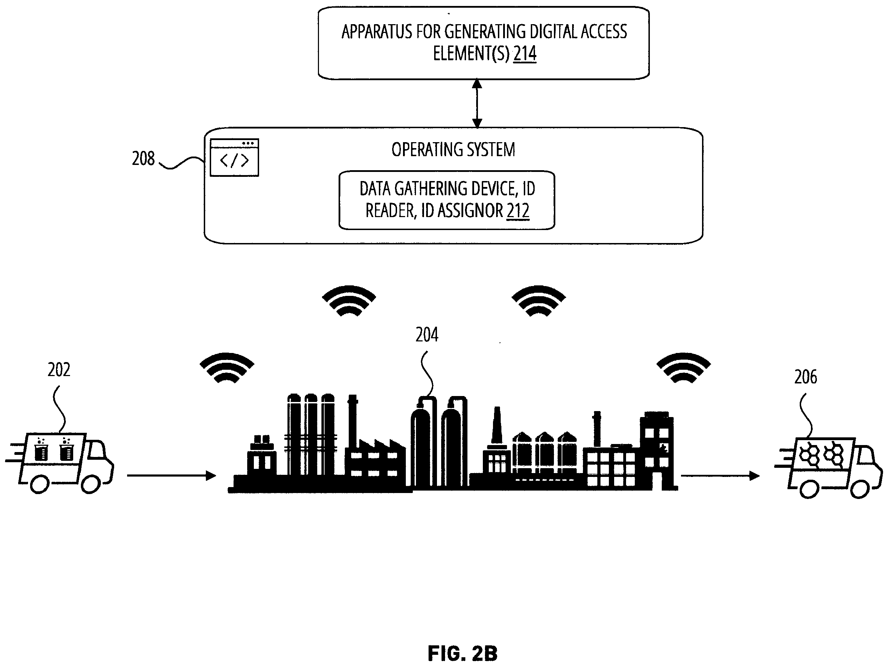

FIG. 2B illustrates another example of a polystyrene composition production 204 controlled by an operating system 208 containing an apparatus for generating a digital access element associated with a polystyrene composition.

The process steps described in the context of FIG. 2A may be executed via an operating system 208 of the polystyrene composition production 204 in interaction with the apparatus for generating digital access element(s) 214 associated with polystyrene composition(s) produced by the polystyrene composition production 204. In this embodiment, the operating system 208 may be communicatively connected to the polystyrene composition production 204 and said apparatus 214. The apparatus may be configured to generate digital access element(s) associated with produced polystyrene composition(s), for example as described in FIG. 2A, FIG. 3 and FIG. 6

The data gathering device of operating system 208 may be configured to gather polystyrene composition data associated with the produced polystyrene composition 206 as described in relation to FIG. 2A. The ID reader of operating system 208 may be configured to read the physical identifier element attached to the packaging of the produced polystyrene composition 206 as described in relation to FIG. 2A. The ID assignor of operating system 208 may be configured to assign the digital identifier provided by apparatus 214 to the physical identifier associated with the produced polystyrene composition 206 as described in relation to FIG. 2A. The data gathering device, the ID assignor and the ID reader 212 and/or the apparatus 214 may be configured as decentral services or applications executed via the decentral network.

FIG. 2A and FIG. 2B only show two example embodiments and any combination of the system components shown in FIG. 2A and FIG. 2B may be possible. For instance, the ID reader may be configured as part of the operating system 208, while the ID provider and the ID assignor may not be configured as part of the operating system 208.

FIG. 3 illustrates an example of an apparatus and associated methods for generating a digital access element in connection with a polystyrene composition produced by a polystyrene composition production.

The polystyrene composition production 204 may produce polystyrene composition(s) 206 from one or more inbound materials (also denoted as precursor materials) 202. The polystyrene composition production 204 may be a chemical production network as described in relation to FIG. 2A. The inbound material(s) 202 may enter the system boundary 302 of the polystyrene composition production 204. The polystyrene composition(s) 206 may be produced using the inbound material(s) 202. The polystyrene composition(s) 206 may exit the system boundary 302 of the polystyrene composition production 204.

Upon producing the polystyrene composition 206 or upon exiting of the polystyrene composition 206 of the polystyrene composition production 204, the digital access element(s) associated with produced polystyrene composition(s) 206 may be generated. The digital access element(s) may be generated by an apparatus for generating digital access element(s) 306. The apparatus 306 may be configured to generate the digital access element(s). The apparatus 306 may be configured to receive a request to provide the decentral identifier. The apparatus 306 may be configured to generate - in response to the received request - the digital access element. A requestor 304 may be configured generate the request for the digital identifier. Said request may be triggered by a labelling system such as a QR Code generator. The request to provide the digital identifier may be provided to a digital ID generator 308 configured to generate the digital identifier. The digital ID generator 308 may provide the generated digital identifier to a digital ID provider 310. While the digital ID generator 308 and the digital ID provider 310 are shown in FIG. 3 as separate units, their functions may be combined within a single unit such that the apparatus 306 comprises a digital ID providing unit configured to generate the digital ID and to provide the generated digital ID.

The digital ID provider 310 may provide the digital identifier to the requestor 304. The requestor 304 may be configured to associate the received digital identifier with the produced polystyrene composition 206. The requestor 308 may hence contain an ID assignor as described in relation to FIG. 2A. Such association may include encoding the digital identifier into a code and providing the code for labelling the polystyrene composition. This way a physical identifier may be provided that relates the physical entity of the polystyrene composition with the provided digital identifier.

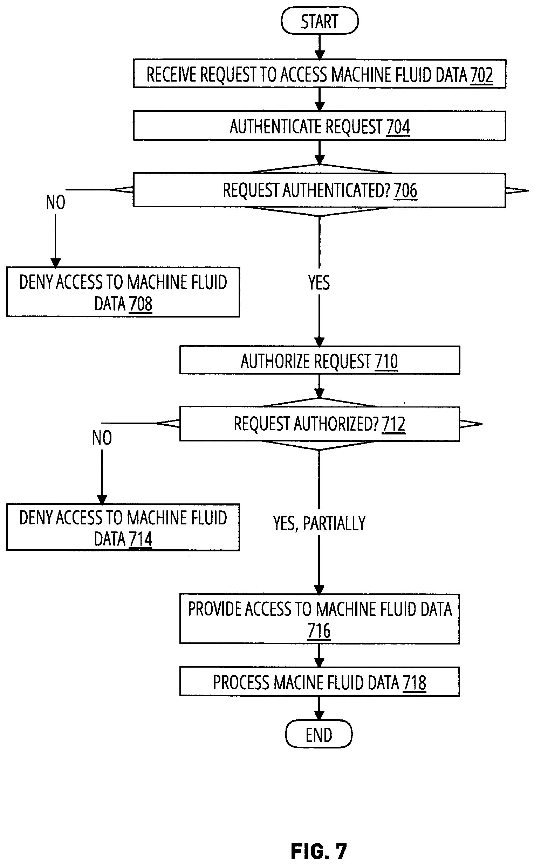

The digital ID provider 310 may provide the digital identifier to a digital access element generator 312 configured to generate the digital access element based on the digital identifier received from digital ID provider 310 and access data. The access element generator 312 may generate the digital access element as described for example in the context of FIG. 4 to FIG. 6. The generated digital access element may include the digital identifier and access data. Access data may include one or more digital representation(s) pointing to polystyrene composition data or parts thereof. The digital access element may include or be related to one or more authentication mechanisms associated with the digital identifier and/or the access data. The authentication mechanisms may be used as described for example in the context of FIG. 7 to FIG. 9. The digital access element may relate to one or more authorization mechanisms associated with the digital identifier and/or the access data. The authorization mechanisms may be used as described for example in the context of FIG. 7 to FIG. 9.

The generated digital access element may be provided to a digital access element provider 314. The digital access element provider 314 may be configured to provide the digital access element for access by a data consuming service 318. The digital access element provider 314 may control the access by the data consuming service 31. The digital access element provider 314 may be a data providing service associated with the polystyrene composition production 204. The digital access element provider 314 may be associated with or under control of a data owner of the polystyrene composition data associated with the generated digital access element. The digital access element may be used to access polystyrene composition data as for example described in the context of FIG. 7 to FIG. 9.

FIG. 4 shows schematically and exemplarily a relation of a digital access element to further data structures in an embodiment. The digital access element may be associated with a polystyrene composition. The digital access element may be associated with a precursor material used to produce the polystyrene composition. The digital access element may be associated with a component, component assembly or end product produced using the polystyrene composition.

The digital identifier may be a decentralized ID abbreviated by DID. In this case, the digital access element may be a DID document associated with the DID.

Besides the DID document serving as digital access element, FIG. 4 shows a DID owner data element 402 including DID-based owner data. The DID owner data element 402 may include a DID associated with a subject. The DID subject may be a precursor material used to produce the polystyrene composition, the polystyrene composition itself or a component, a component assembly or an end product produced using the polystyrene composition. The DID subject may be a machine, a system, or a device used for transporting or producing the precursor material, the polystyrene composition itself or a component, a component assembly or an end product produced using the polystyrene composition, or a collection of such machine(s), device(s) and/or system(s). The DID owner data element 402 may include authentication mechanisms. The DID owner data element 402 may include owner data that is electronically owned and controlled by the DID owner. In this context electronically owned may refer to data that is stored in an owner repository or wallet. Such data may be securely stored and/or managed on an organizational server or client device. The owner repository or wallet may be associated with or under control of the data owner of the polystyrene composition data. The DID owner data element may include a DID, a private key and a public key.

The DID owner may own and control the DID that represents an identity associated with the DID subject, a private key and public key pair that are associated with the DID. DID may be understood as an identifier and authentication information associated with or uniquely linked to the identifier. The DID owner may be a supply chain participant or a manufacturer such as a polystyrene composition producer. The DID owner may be an upstream participant in the supply chain of the polystyrene composition producer such as a supplier that supplies precursor materials to produce polystyrene composition s. The DID owner may be a downstream participant in the supply chain of the polystyrene composition such as a customer that consumes polystyrene composition to produce the component, the component assembly or the end product. The DID owner may be any participant of the supply chain including raw chemical product supplier, intermediate chemical products manufacturer, intermediate part manufacturer, component manufacturer, component assembly manufacturer or end product manufacturer.

The DID may be any identifier that is associated with the DID subject and/or the DID owner. Preferably, the identifier is unique to the DID subject and/or DID owner. The identifier may be unique at least within the scope in which the DID is anticipated to be in use. The identifier may be a locally or globally unique identifier for the above mentioned DID subjects. The DID may be a Uniform Resource Identifier (URI) such as a Uniform Resource Locator (URL). The DID may be an Internationalized Resource identifier (IRI). The DID may be a random string of numbers and letters for increased security. In one embodiment, the DID may be a string of 128 letters and numbers e.g. according to the scheme did:method name: method specific-did such as did:example:ebfeb1f712ebc6f1c276e12ec21. The DID may be decentralized, independent of a centralized, third-party management system and under the control of the DID owner.

The digital access element as DID document may be associated with the DID. The DID document may include DID document data 404. DID document data 404 may include the DID. Accordingly, the digital access element may include a reference to the DID, which may be associated with the DID subject that is described by the DID document. The DID document data 404 may include access data. Access data may include the public key of the DID owner/subject. The public key may be used by third-party entities that are given permission by the DID own-er/subject to access information and data owned by the DID owner/subject. The public key may be used for verifying that the DID owner, in fact, owns or controls the DID. Access data may include authentication information, authorization information e.g. to authorize third party entities to read the DID document or some part of the DID document e.g. without giving the third party the right to prove ownership of the DID.

Access data may include one or more representations that digitally link to the polystyrene composition data, e.g. by way of service endpoints. A service endpoint may include a network address at which a service operates on behalf of the DID owner. In particular, the service endpoints may refer to services of the DID owner that give access to polystyrene composition data. Such services may include services to read or analyze polystyrene composition data. Additionally or alternatively to the possible types of polystyrene composition data indicated already, the polystyrene composition data may include declaration data, safety data, certificate of analysis data, emission data, product carbon footprint data, product environmental footprint data, specification data, product information, technical application data, production data, transport data, or combinations thereof.

The DID document data may include various other information such metadata specifying when the DID document was created, when it was last modified and/or when it expires.

The DID and DID document may be associated with a retrieval data storage in the form of a data registry. The data registry may be a centralized data service system or a decentralized data service system, e.g. a distributed ledger, a blockchain or a distributed file system 406, 408. Possible blockchain systems include Quorum, Hyperledger Fabric, or the like. The distributed ledger, blockchain or distributed file system 406, 408 may be used to store a representation of the DID pointing to the DID document. A representation of the DID may be stored on distributed computing nodes of the distributed ledger, blockchain or distributed file system 406, 408. For example, DID hash may be stored on multiple computing nodes of the distributed ledger, blockchain or distributed file system 406, 408 and point to the location of the DID document. In some embodiments, the DID document may be stored on the distributed ledger, blockchain or distributed file system 406, 408. Alternatively, in other embodiments the DID document may be stored in a data storage (not illustrated) that is associated with the distributed ledger, blockchain or distributed file system 406, 408.

The distributed ledger, blockchain or distributed file system 406, 408 may be any decentralized, distributed network that includes various computing nodes that are in communication with each other. For example, the distributed ledger, blockchain or distributed file system 406, 408 may include a first distributed computing node, a second distributed computing node, a third distributed computing node, and any number of additional distributed computing nodes. The distributed ledger, blockchain or distributed file system 406, 408 may operate according to any known standards or methods for distributed ledgers. Examples of conventional distributed ledgers that correspond to the distributed ledger or blockchain 406, 408 include, but are not limited to, Bitcoin [BTC], Ethereum, and Litecoin.

FIG. 5 shows schematically and exemplarily a relation of a digital access element to further data structures in a further embodiment. The digital access element may be associated with a polystyrene composition. The digital access element may be associated with a precursor material used to produce the polystyrene composition. The digital access element may be associated with a component, component assembly or end product produced using the polystyrene composition.

In contrast to the example of FIG. 4, the example of FIG. 5 is certificate based, i.e. based on certificate data 502 instead of a data structure like a DID owner data element 402. The ID-based certificate data 502 may include authentication data of the certificate owner and the certificate issuer. For example, a cryptographic signature from the issuer may bind the public key of the data owner to the ID. The ID may be a unique ID (such as UID) as described in relation to the DID of Figure 2. The certificate may be a X.509 certificate such as X509v3.

The digital access element is in this case an ID-based passport including ID-based passport data 504, which may be associated with a data source of the data owner. The ID-based passport data 504 may include an ID, authentication data and endpoints associated with the polystyrene composition data. Such endpoints may include any digital representation connecting to the data source. The data source may provide the polystyrene composition data.

In this certificate-based example, the ID-based passport data 504 may include one or more certificate(s) associated with the data owner. The certificates may be associated with an identity manager including e.g. a certificate issuing service and/or a dynamic provisioning service providing dynamic attribute tokens (e.g. OAuth Access Tokens). The information required to verify the certificates may be provided via an authentication registry associated with the certificate issuing service and/or a dynamic provisioning service. For instance, in the IDSA Reference Architecture Model, Version 3.0 of April 2019, a connector associated with the data owner, a Certification Authority (CA) 510, a Dynamic Attribute Provisioning Service (DAPS) 512 and a connector associated with the data consuming service are used to verify the identity prior to performing a data exchange (not shown). For this purpose, such connectors may include one or more certificate(s) such as X.509 certificate(s). This way the connector possesses a unique identifier embedded in a X.509 certificate that identifies the connector instance.

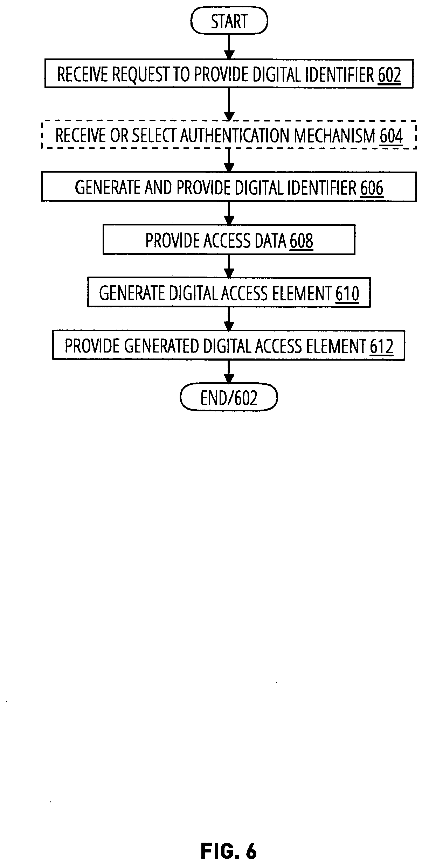

FIG. 6 shows an example method for generation of a digital access element. The digital access element may be associated with a polystyrene composition. The digital access element may be associated with a precursor material used to produce the polystyrene composition. The digital access element may be associated with a component, component assembly or end product produced using the polystyrene composition.

The method may be performed by the apparatus for generating digital access element(s) 306 described in relation to FIG. 2A, FIG. 2B and FIG. 3. For digital access element generation, a request to provide a digital identifier associated with a polystyrene composition and polystyrene composition data may be provided. The request may be provided by a requestor 308 described in relation to FIG. 3. A computing node (that acts as a DID owner's management module, user agent, ID hub and/or certification issuer) may receive the request to provide the digital identifier 602. The computing node may correspond to the digital ID generator 308 of FIG. 3. The request may contain the format of the identifier, such as DID or UUID. The request may contain at least one authentication mechanism.

The computing node may receive at least one authentication mechanism or may select at least one of multiple authentication mechanisms based on data contained in the received request (see block 604). The at least one authentication mechanism may need to be provided or selected depending on the type of digital identifier. Hence, block 604 may be optional for certain types of digital identifiers, such as digital identifiers based on UUID standard.

In block 606, the digital identifier may be generated and provided. The digital identifier may be provided by the node generating the digital identifier (see also FIG. 3). The digital identifier maybe provided by a further node in communication with the node generating the digital identifier (see also FIG. 3). Along with the digital identifier, data related to the authentication mechanism associated with the digital identifier may be provided.

In block 608, access data may be provided. Access data may include a digital representation pointing to polystyrene composition data or parts thereof. Access data may include the data mentioned previously, such as in relation to FIG. 2A, FIG. 2B and FIG. 3. Access data may be provided by the node generating and/or providing the digital identifier.

Based on the provided digital identifier and the provided access data, the digital access element may be generated (see block 610). The digital access element may be generated by a digital access element generator 312 (see FIG. 2A, FIG. 2B and FIG. 3). The digital access element may contain the digital identifier and access data. The digital access element may further contain data related to the authentication mechanism associated with the digital identifier and/or with the digital access element.

The generated digital access element may be provided for access by a data consuming service, for example by a digital access element provider 314. The access may be controlled by the digital access element provider 314. The digital access element provider may be associated with a data owner, such as a data owner of the polystyrene composition data associated with the digital identifier. The data owner may operate the polystyrene composition production producing the polystyrene composition.

At least a portion of data contained in the digital access element may be propagated to an authentication data registry (e.g. retrieval data storage) such as a distributed ledger, a distributed database, a distributed file system or a central database (see for example FIG. 4).

FIG. 7 shows an example method for using the digital access element to further process the polystyrene composition associated with the digital access element.

For using the digital access element, an indication to access the polystyrene composition data associated with a digital identifier of the digital access element may be received (block 702). The digital access element may be structured as lined out in FIG. 4 and FIG. 5. The digital access element may be generated as lined out in FIG. 2A, FIG. 2B. FIG. 3 and FIG. 6.

Before access may be provided to the polystyrene composition data, the request may be authenticated (blocks 704, 706). In particular, the data consuming service requesting to access to the polystyrene composition data and/or the data providing service providing access to the polystyrene composition data may be authenticating. Such authentication may be based on the decentral identity and the data related to the authentication mechanism. The authentication may be performed through different communication patterns.

If the authentication fails, access to the polystyrene composition data may be denied (block 708). If the authentication is valid, an authorization step may follow (block 710). Such authorization may be based on the decentral identity and the data related to the authorization rules. The authorization may be performed through different communication patterns.

If the authorization fails, access to the polystyrene composition data may be denied (block 714). If authorization partly fails access may be adapted. In particular, the authorization as requested may be adapted to be in line with the applicable authorization rules. If the authorization is valid, access to the polystyrene composition data may be granted according to the authorization rules as requested (block 716). Such access to polystyrene composition data associated with the decentral identifier may be provided using the representation embodied in the digital access element.

The accessed polystyrene composition data may be processed (block 718). Processing may be performed according to applicable authorization rules. Processing may include determining recycling instructions based on the recycling instruction data contained in the accessed polystyrene composition data. For instance, a recycling location or recycling operation may be determined based on the accessed polystyrene composition data. The recycling instructions may be determined for polystyrene composition residues generated during installation of the polystyrene composition, such as extruded polystyrene boards or materials produced from expanded polystyrene. Processing may include determining installation instructions based on the accessed polystyrene composition data.

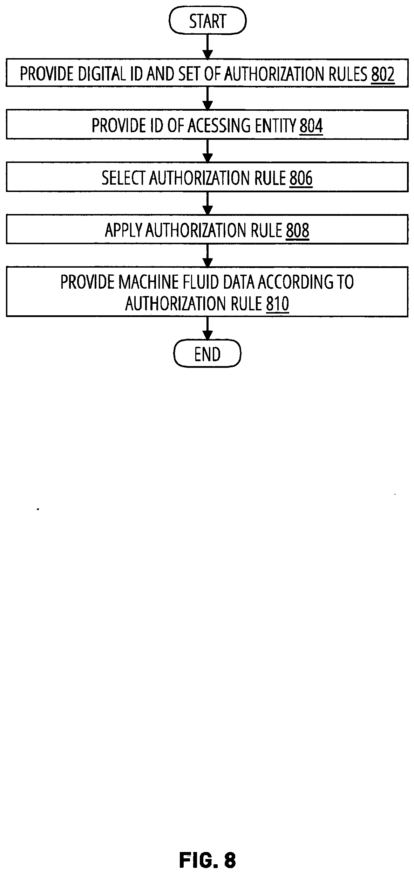

FIG. 8 shows an example method for authorizing access to polystyrene composition data.

In block 802, a digital identifier (denoted as polystyrene composition identifier hereinafter) and a set of authorization rules for the polystyrene composition data associated with the digital identifier may be provided. The set of authorization rules may include usage instructions defining usage policies for entities accessing the polystyrene composition data associated with the digital identifier. The set of rules may include one or more local rules that are specific to a particular location. The one or more local rules may be based on a location of where the digital identifier was generated, where the data providing service was implemented, where the data consuming service was implemented or a combination thereof.

The one or more sets of local rules may be based on a location provided by the data providing service or the data consuming service. The location may refer to a jurisdiction and the local rules may be associated with legal requirements related to the supply of polystyrene composition(s). For instance, access to polystyrene composition data may be provided via an authorization rule that may include jurisdictional or local rules. Polystyrene composition data may include certificate of analysis data associated with precursor materials and/or the polystyrene composition itself. Polystyrene composition data may include product safety data associated with hazards of substances or mixtures of the physical entity of the polystyrene composition. Polystyrene composition data may include product declaration data associated with the physical entity of the precursor material(s) and/or the polystyrene composition(s). Polystyrene composition data may include the data mentioned previously.

In block 804, a further digital identifier (denoted as accessing entity identifier hereinafter) or data related to the further digital identifier of the accessing entity may be provided. Based on the accessing entity identifier or data related to the accessing entity identifier, the authorization rule(s) for the polystyrene composition data associated with the polystyrene composition identifier may be selected. The authorization rule(s) may include computer-executable instructions to allow access to, deny access to or modify polystyrene composition data. The authorization rule(s) may relate to each data point of the polystyrene composition data or sets or classes of polystyrene composition data. The selected authorization rule(s) may be stored for application to the polystyrene composition data.

In block 808, the selected authorization rule(s) may be applied before or on data transaction.
The selected authorization rule(s) may be bound to the polystyrene composition data, individual data points or classes of polystyrene composition data for application to the polystyrene composition data. The polystyrene composition may include data related to different classes of polystyrene composition data. For instance, the polystyrene composition data may itself include different classes of polystyrene composition data as previously described. In one embodiment at least one class of polystyrene composition data includes data required by regulation or regulatory data for polystyrene composition.

The selected authorization rule(s) may be applied to the polystyrene composition data associated with the polystyrene composition identifier. The selected authorization rule(s) may be applied prior to access of the polystyrene composition data. The selected authorization rule(s) may be applied during run-time on access of the polystyrene composition data.

In block 810, the polystyrene composition data associated with the polystyrene composition identifier may be provided according to the selected authorization rule(s). The authorization rule(s) may for instance may include local rules that are specific to a particular location, wherein the location is associated with a jurisdiction and the local rule for the location is associated with legal requirements related to the supply of polystyrene composition. The set of authorization rules may include at least one regulatory instruction configured to provide access to polystyrene composition data relating to regulatory requirements for the supply of polystyrene composition. The provided set of authorization rules may be related to decentral identifiers of accessing entities. the authorization rule may include computer-executable instructions to allow access to polystyrene composition data associated with the polystyrene composition identifier, deny access to polystyrene composition data associated with the polystyrene composition identifier and/or to modify access to polystyrene composition data associated with the polystyrene composition identifier. The authorization rule(s) may relate to each data point of the polystyrene composition data or classes of polystyrene composition data, wherein the selected authorization rule(s) may be bound to the polystyrene composition data, classes of chemical polystyrene composition, individual data points or combinations thereof. The set of authorization rules may include one or more of prescribed rules relating to obligations of the data consuming service associated with the accessing entity identifier. The set of authorization rules may include one or more of prescribed rules relating to emission data, production data, recycled content data, bio-based content data, provenance data, labor conditions data or combinations thereof. The set of authorization rules may include one or more processing rules relating to the processing of emission data, production data, recycled content data, bio-based content data, provenance data, labor conditions data or combinations thereof by a data consuming service associated with decentral identifiers of accessing entities. The set of authorization rules may include one or more aggregation rules relating to bill of material data provided by a data providing service of a supplier or precursor material data provided by a data providing service of a supplier.

FIG. 9 illustrates schematically an example of a method or apparatus for providing polystyrene composition data associated with polystyrene composition(s) to a polystyrene composition consumer as data consumer via a decentral network.

The polystyrene composition(s) 206 as produced by the polystyrene composition production 204 may be provided in association with the digital access element as described in the context of FIG. 2A, FIG. 2B, FIG. 3 and 6. The digital access element may include the digital identifier. The digital access element may include access data. The digital access element may be associated with polystyrene composition data.

The digital access element may further include or relate to authentication and/or authorization information linked to the digital identifier. The authentication and/or authorization information may be provided for authentication and/or authorization of a data providing service 902 and/or data consuming service 318. The digital identifier may include or relate to a decentral identifier that is uniquely associated with the polystyrene composition. The decentral identifier may be connected to the access data. The access data may include a representation for accessing the polystyrene composition data or parts thereof. The decentral identifier may include a Universally Unique IDentifier (UUID) or a Digital IDentifier (DID). The decentral identifier may include any unique identifier uniquely associated with a data owner and/or the polystyrene composition. The data owner may be the producer of the polystyrene composition(s). The data owner may comprise any entity generating data. The data generating node may be coupled to the data owner or the entity owning or producing polystyrene composition(s) from or for which data is generated. The data may be generated by a third-party entity on behalf of the entity owning polystyrene composition(s) from or for which data is generated. Via the decentral identifier and its unique association with the data owner and/or the polystyrene composition(s), access to the polystyrene composition data may be controlled by the data owner.

The digital access element including the access data may be stored in a decentral data base 904. The polystyrene composition data associated with the digital identifier may be stored in a data base 906 associated with the data owner, such as the producer of the polystyrene composition(s) 206.

The polystyrene composition(s) 206 may be physically delivered to a consumer of the polystyrene composition(s). The polystyrene composition may be connected with a code or having embossed a code having encoded the digital identifier. The consumer of the polystyrene composition may read the code through a code reader, such as a QR-code reader, 910. The digital identifier may be provided to a data base 908 associated with the consumer of the polystyrene composition 206. In other embodiments the consumer of the polystyrene composition may retrieve the digital identifier through the decentral data base 904.

Based on the received digital identifier, a request to access the polystyrene composition data associated with the digital identifier may be triggered by the data consuming service 318 as signified by arrow 912. The digital identifier may be provided to the data providing service 902 associated with or of the producer of the polystyrene composition 206.In addition, authentication and/or authorization information may be provided.

The request may be authenticated and/or authorized to access the polystyrene composition data associated with the digital identifier, for instance as described in the context of FIG. 7 and FIG. 8. Based on successful authorization and/or authentication, access to the polystyrene composition data associated with the digital identifier may be granted.

For access, the digital identifier may be provided to the data providing service 902 as signified by arrow 912. The data providing service 902 may use the received digital identifier to retrieve the polystyrene composition data associated with the received digital identifier as signified by arrows 914 and 916. The retrieved polystyrene composition data associated with received digital identifier may be provided to the data consuming service 318 as signified by arrow 918. Authorization rule(s) may be applied to the retrieved polystyrene composition data as described in the context of FIG. 8. The polystyrene composition data associated with the polystyrene composition 206 may be stored in the data base 908 associated with the consumer of the polystyrene composition 206 as signified by arrow 920.

Through the digital identifier, the polystyrene composition data can be uniquely associated with the polystyrene composition. Through the decentral network, the polystyrene composition data may be transferred between the producer of the polystyrene composition the consumer of the polystyrene composition. This way the polystyrene composition data can be shared with unique association to the polystyrene composition and without central intermediary directly between the value chain players. This allows for simplified, secure and controlled sharing of polystyrene composition data between value chain players.

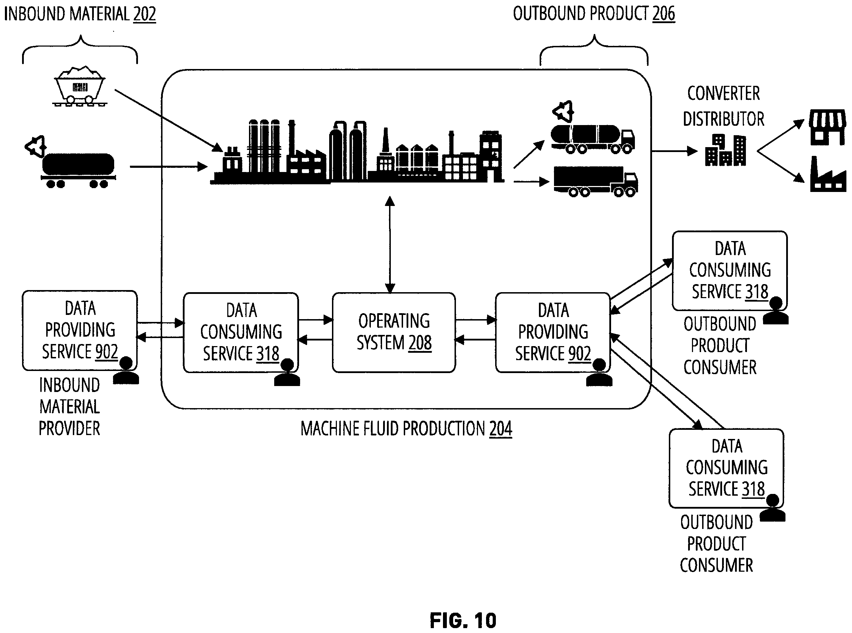

Fig. 10 illustrates schematically an example of a method or apparatus for polystyrene composition data associated with a polystyrene composition across value chains via the decentral network.

In the example of Fig. 10, a fully connected value chain including the polystyrene composition production 204 is illustrated. In the example, the inbound material provider, the polystyrene composition producer, the polystyrene composition consumer and the end product producer may be connected to the decentral network as described in the context of Fig. 9. Polystyrene composition data may be provided via digital access elements described in the context of FIG. 2A, FIG. 2B, FIG. 3 to FIG. 6 associated with the physical entity of the input material, the polystyrene composition, any intermediate product or the end product.

The inbound material provider may provide the inbound material 202. The inbound material 202 may include virgin and/or recycled material, such as recycled ethylene glycol. The precursor material data associated with said inbound material 202 may be provided through the data providing service 902 connected to the decentral network as described in the context of FIG. 9. The polystyrene composition producer may produce the polystyrene composition from the inbound material(s) 202 provided to the polystyrene composition production 204. The polystyrene composition producer may access the precursor material data associated with the inbound material 202 through a data consuming service 318 connected to the decentral network as described in the context of FIG. 9. Such precursor material data may be retrieved from a data providing service 902 associated with the respective inbound material provider. The polystyrene composition producer may generate digital access element for the produced polystyrene composition(s) as described in the context of FIG. 2A, FIG. 2B, FIG. 3 and 6.

The polystyrene composition producer may provide the polystyrene composition data associated with the produced polystyrene composition(s) 206 through the data providing service 902 connected to the decentral network as described in the context of FIG. 9. The polystyrene composition consumer or the end product producer may access the polystyrene composition data associated with the produced polystyrene composition(s) 206 through the data consuming service 318 connected to the decentral network as described in the context of FIG. 9. The produced polystyrene composition may be provided to converters, such as component assembly producers, which may in turn may supply the produced products to traders and/or further customers, such as end users or industrial customers. The produced polystyrene composition may be provided to distributors, which in in turn may supply the received polystyrene composition to traders and/or further customers, such as end users or industrial customers.

The respective data owners in this example may be the input material producer, polystyrene composition producer, the polystyrene composition consumer, the end product producer. The data owner may comprise any entity generating data. The data generating node may be coupled to the data owner or the entity owning or producing physical products from or for which data is generated. The data may be generated by a third-party entity on behalf of the entity owning physical products from or for which data is generated.

In the example of FIG. 10, the digital identifier may relate to the end product. Such digital identifier may be provided to the value chain participants. Via the end product specific digital identifier, data associated with the end product produced from the polystyrene composition may be gathered across the production chain and assigned to the end product specific digital identifier. For instance, recycled content contained in the polystyrene composition, such as the content of recycled ethylene glycol contained in the engine coolant, may be derived from the polystyrene composition data associated with the polystyrene composition.

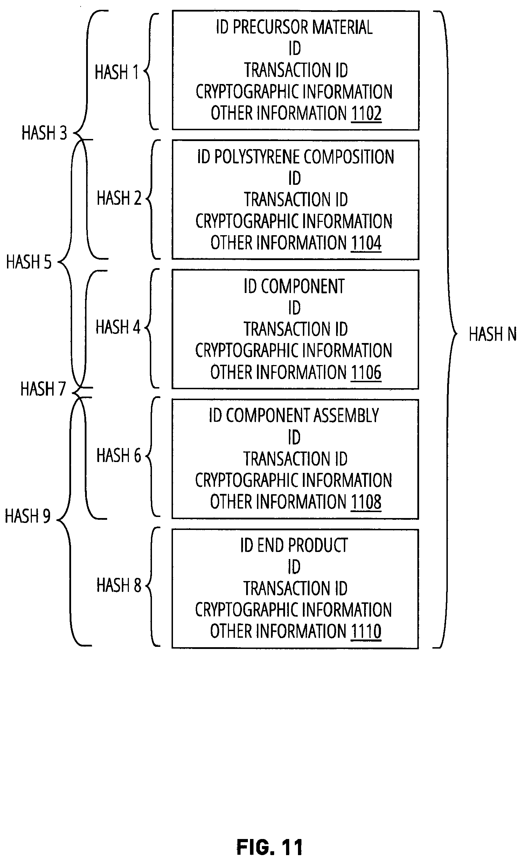

FIG. 11 to FIG. 16 show different example configurations for digital access elements anchored by digital identifiers. The configurations may include different parent, child, grandchild and so on relationships for digital access elements generated in a chemical value chain up to an end product. The chemical value chain may include polystyrene composition s, such as engine coolants and/or brake fluids.

FIG. 11 illustrates an individual configuration for different digital access elements generated in the chemical value chain. For multiple product stages in the chemical value chain individual digital access elements may be generated, for example as described in the context of FIG. 3 and FIG. 6. Among these digital access elements, a digital access element associated with a polystyrene composition may be generated. The digital access element generation may include the providing of a digital, in particular a decentral, identifier and access data indicating a required authentication mechanism or service endpoint to access the data for each of the multiple product stages. The digital access elements for the multiple product stages may be based on crypto signatures as part of the access data. For instance, the digital access elements for the multiple product stages may be concatenated through hash values based on different data sets. As shown in Fig. 11, hash 1 may be based on data of the digital access element associated with the precursor material ("precursor material digital access element"), hash 2 may be based on data of the digital access element associated with the polystyrene composition ("polystyrene composition digital access element") and hash 3 may be based on data of the precursor material digital access element plus data of the polystyrene composition digital access element. Further concatenation may be done for other combinations of digital access elements up to hash n, which concatenates digital access elements up to the end product passport. Concatenation via hashes of the crypto signature is only one example concatenation. Other examples include permission aggregations with different scope of data that may be embedded in child passports, public key aggregations with different crypto signatures or service endpoint aggregation with different links.

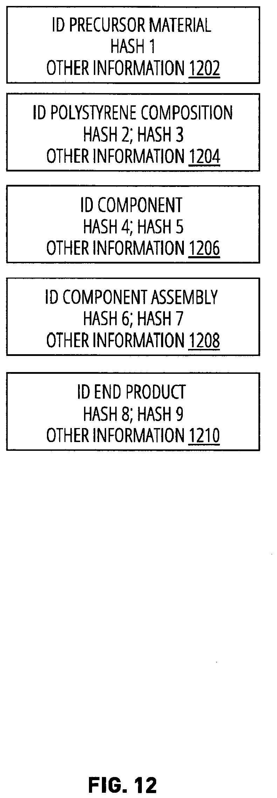

Fig. 12 illustrates different digital access elements including a concatenation associated with multiple digital identifiers based on the relationship representation for different products associated with product stages of the chemical value chain. Among the digital access elements, one digital access element is associated with a polystyrene composition. In this particular example, hash values are used for the concatenation associated with multiple digital identifiers. The data sets to generate the hash values are schematically illustrated in Fig. 11.

The precursor material digital access element may be provided to the polystyrene composition producer using the precursor material to produce the polystyrene composition. The precursor material digital access element may be connected to a hash value hash 1. The hash value hash 1 may be generated via a hashing algorithm such as MD5, SHA-1, SHA-2, SHA-3 or any other suitable algorithm based on a one-way function that can't be reverse engineered. The hash value hash 1 may be generated based on data included in or connected to the precursor material digital access element. The data for hash generation may include the digital identifier and the access data. The data for hash generation may include the precursor material data. The data for hash generation may include the digital identifier associated with the precursor material, the access data, the precursor material data associated with the precursor material and/or cryptographic information connected to the digital identifier. The hash value hash 1 may be used by participant nodes of the chemical supply chain to check integrity of the data package transferred from the precursor material supplier e.g. to the polystyrene composition producer.

Similar to the precursor material digital access element, the polystyrene composition digital access element may be provided to the component producer using the polystyrene composition to produce the component, such as a machine or a part thereof. The generation of the polystyrene composition digital access element may be based on the precursor material digital access element provided to the polystyrene composition producer using the precursor material to produce the polystyrene composition. The polystyrene composition digital access element may be connected to one or more hash value(s) hash 2, hash 3. The hash values hash 2, hash 3 may be generated via a hashing algorithm such as MD5, SHA-1, SHA-2, SHA-3 or any other suitable algorithm based on a one-way function that can't be reverse engineered. The hash values hash 2, hash 3 may be generated based on data included in or connected to the polystyrene composition digital access element and/or the precursor material digital access element. The hash value(s) hash 2, hash 3 may be generated based on clear data itself or based on hash value(s) generated from the clear data. For instance, hash 3 may be generated based on digital access element data or based on hashed digital access element data. The data for hash generation may include the digital identifier associated with the precursor material used to produce the polystyrene composition, the digital identifier associated with the polystyrene composition, the access data associated with the precursor material digital access element and/or the access data associated with the polystyrene composition digital access element.

The concatenation associated with multiple digital identifiers may relate to the digital identifiers associated with the polystyrene composition and the precursor material(s). Hashing data related or included in the respective digital access elements may provide for such concatenation. The data for hash generation may include the precursor material data and/or the polystyrene composition data. The data for hash generation may include the digital identifier associated with the precursor material, the access data, the precursor material data associated with the precursor material and/or cryptographic information connected to the digital identifier. The data for hash generation may include the digital identifier associated with the polystyrene composition, the access data, the polystyrene composition data associated with the polystyrene composition and/or cryptographic information connected to the digital identifier. Hash value hash 2 may be generated in relation to the polystyrene composition digital access element as illustrated in Fig. 11. The hash value hash 2 may be based on at least the digital identifier associated with the polystyrene composition and the access data. Hash value hash 3 may be generated in relation to the precursor material digital access element and the polystyrene composition digital access element as illustrated in Fig. 11. The hash value hash 3 may be based on at least the digital identifier associated with the precursor material, the access data associated with the precursor material digital access element, the digital identifier associated with the polystyrene composition and the access data associated with the polystyrene composition digital access element. The hash value(s) may be used by participant nodes of the chemical supply chain to check integrity of the data package transferred from the precursor material supplier or the polystyrene composition producer. The combined hash value(s) may further be used by participant nodes of the chemical supply chain to determine the relation of products at different stages and to check integrity of such relation.

As illustrated in Figs. 11 and 12, the hash value(s) may be connected to the digital access elements associated with one or more product stage(s) of the chemical supply chain.

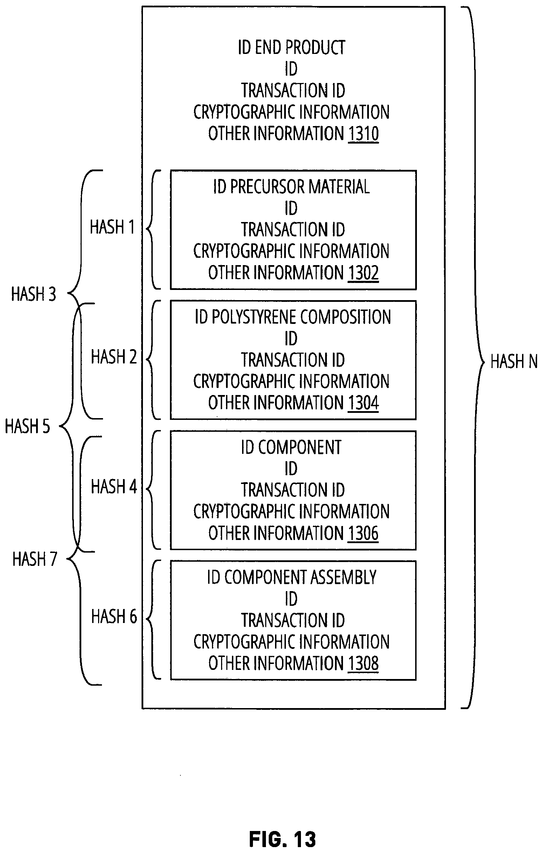

Fig. 13 illustrates an anchored configuration for different digital access elements generated in the chemical value chain. For the end product an end product digital access element is generated. For multiple further product stages in the chemical value chain individual digital access element may be generated, among them the polystyrene composition digital access element. The individual digital access elements may be embedded in or linked with the end product digital access element. The digital access element generation may include the providing of a digital identifier and access data indicating a required authentication mechanism and/or service endpoint to access the respective data for each of the multiple product stages. The digital access elements for the multiple product stages may be based on crypto signatures as part of the access data. For instance, the digital access elements for the multiple further product stages may be concatenated through hash values based on different data sets, specifically the different data included in the respective digital access elements. As shown in Fig. 13, hash 1 may be based on data of the precursor material digital access element, hash 2 may be based on data of the polystyrene composition digital access element and hash 3 may be based on data of the precursor material digital access element plus data of the polystyrene composition digital access element. Further concatenation may be done for other combinations of digital access elements up to hash n, which concatenates digital access elements up to the end product digital access element. Concatenation via hashes of the crypto signature is only one example concatenation. Other examples include permission aggregations with different scope of data that may be embedded in child digital access element, public key aggregations with different crypto signatures or service endpoint aggregation with different links.

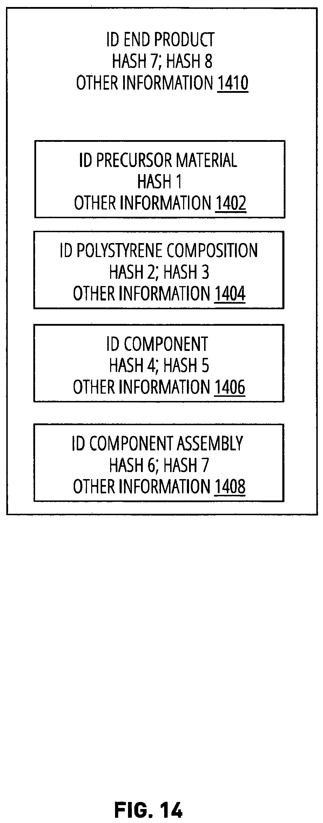

Fig. 14 illustrates different digital access elements including a concatenation associated with multiple digital identifiers based on a relationship representation for different products associated with product stages of the chemical value chain. Among the digital access elements, one digital access element is associated with a polystyrene composition. In this particular example, hash values are used for the concatenation associated with multiple digital identifiers. The data sets to generate the hash values are schematically illustrated in Fig. 13.

The precursor material digital access element may be provided to the polystyrene composition producer using the precursor material to produce the polystyrene composition. The precursor material digital access element may be connected to a hash value hash 1 that may be generated as described in the context of Fig. 13.

Similar to the precursor material digital access element, the polystyrene composition digital access element may be provided to the component producer using the polystyrene composition to produce the component. The hash values may be generated as described in the context of Fig. 12. The hash values may be generated in a similar fashion for the digital access element up to the end product digital access element.

In the anchored configuration, the end product digital access element may include hash values that relate to the digital access element associated with the products used to produce the end product. The concatenation associated with multiple digital identifiers may in this case relate to the digital identifiers associated with the precursor materials(s), the polystyrene composition s(s), the component(s) and the component assembly/ies. Hashing data related or included in the respective digital access elements may provide for the concatenation. The data for hash generation may include any data included in respective digital access elements. The hash value hash 7 may be based on at least the digital identifier associated with the end product and access data associated with the end product digital access element. Hash value hash 8 may be generated in relation to the product digital access element associated with products at different product stages as illustrated in Fig. 13. The combined hash value hash 8 may be used by participant nodes of the chemical supply chain to determine the relation of products at different stages and to check integrity of such relation.

As illustrated in Figs. 13 and 14, the hash value(s) may be connected to the digital access elements associated with one or more product stage(s) of the chemical supply chain.

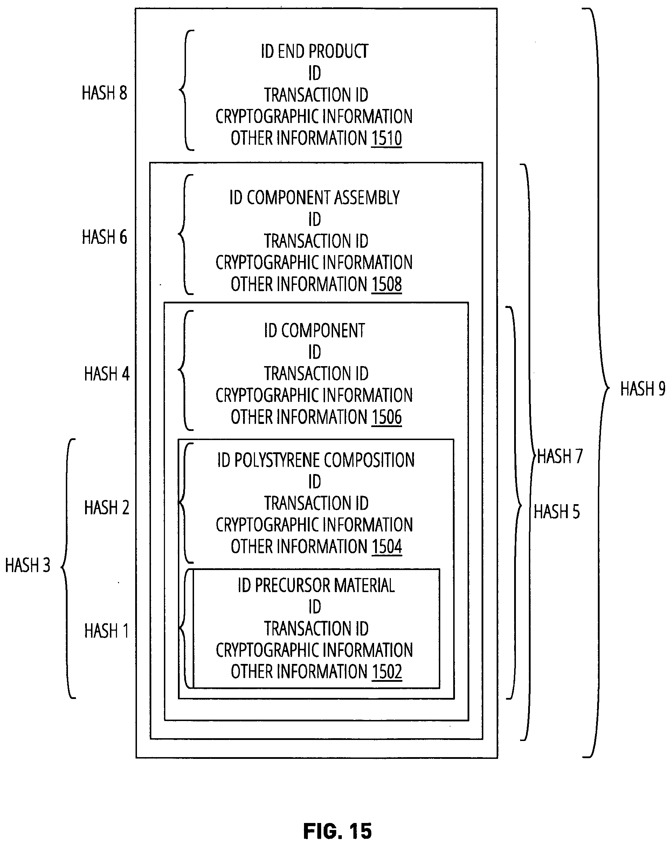

Fig. 15 illustrates a fully embedded configuration for different digital access elements generated in the chemical value chain. For multiple product stages in the chemical value chain individual product passports may be generated, among them the polystyrene composition digital access element. The digital access element may include the providing of a digital identifier and access data indicating a required authentication mechanism and/or service endpoint to access the data for each of the multiple product stages. The digital access elements for the multiple product stages may be based on crypto signatures as part of the access data. For instance, the digital access elements for the multiple product stages may be concatenated through hash values based on different data sets. As shown in Fig. 15, hash 1 may be based on data of the precursor material digital access element. Hash 2 may be based on data of the precursor material digital access element and the polystyrene composition digital access element. Further concatenation may be done for other combinations of digital access elements up to hash n, which concatenates digital access elements up to the end product digital access element. Concatenation via hashes of the crypto signature is only one example concatenation. Other examples include permission aggregations with different scope of data that may be embedded in child digital access elements, public key aggregations with different crypto signatures or service endpoint aggregation with different links.

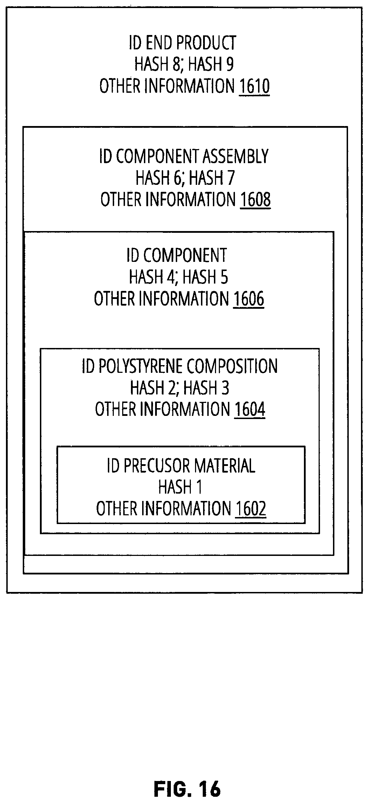

Fig. 16 illustrates different digital access elements including a concatenation associated with multiple digital identifiers based on a relationship representation for different products associated with product stages of the chemical value chain. Among the digital access elements, one digital access element is associated with a polystyrene composition. In this particular example, hash values are used for the concatenation associated with multiple digital identifiers. The data sets to generate the hash values are schematically illustrated in Fig. 15.

The precursor material digital access element may be provided to the polystyrene composition producer using the precursor material to produce the polystyrene composition. The precursor material digital access element may be connected to a hash value hash 1 that may be generated as described in the context of Fig. 15. Similar to the precursor material digital access element, the polystyrene composition digital access element may be provided to the component producer using the polystyrene composition to produce the component. The hash values may be generated as described in the context of Fig. 15. The hash values may be generated in a similar fashion for the digital access elements up to the end product digital access element.

In the fully embedded configuration, the combined hash values may be generated from digital access elements associated with all products preceding the product. The concatenation associated with multiple digital identifiers may in this case relate to the digital identifiers associated with all preceding products, such as the precursor material(s), the polystyrene composition(s), the component(s) and the component assembly/ies. Hashing data related or included in the respective digital access element may provide for the concatenation. The data for hash generation may include any data included in respective digital access elements. The hash value hash 7 for example may be based on at least the digital identifier associated with the products up to the end product and the access data. Hash value hash 9 for example may be based on at least the digital identifier associated with the products up to and including the end product and the respective access data. The combined hash values hash, 3, hash 5, hash 7 and hash 9 may be used by participant nodes of the chemical supply chain to determine the relation of products at different stages and to check integrity of such relation. As illustrated in Figs. 15 and 16, the hash value(s) may be connected to the digital access elements associated with one or more product stage(s) of the chemical supply chain.

The configurations shown in FIGs. 11 to 16 relate to digital access elements generated in the chemical value chain up to an end product. Similarly, digital access elements generated in the recycling chain from end product to recyclate(s) may be concatenated. Further similarly, the digital access elements generated in the chemical value chain up to an end product and in the recycling chain from end product to recyclate(s) may be concatenated. This way the circularity of products and in particular materials may be virtually represented and tracked.

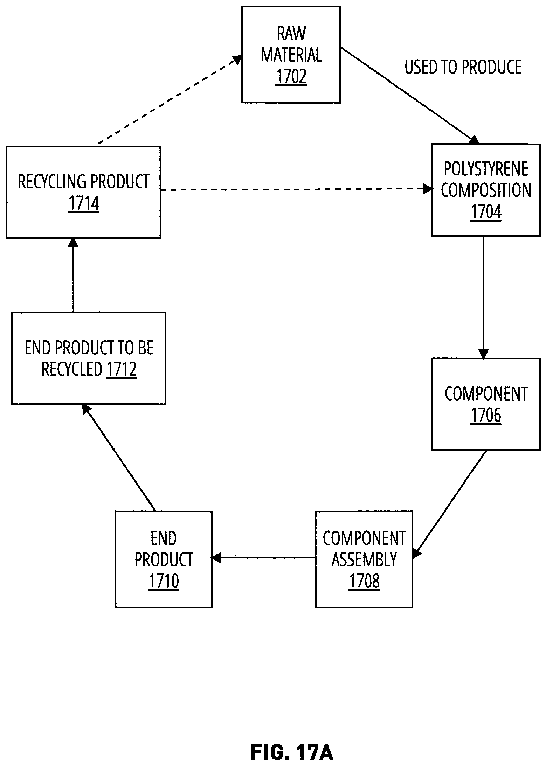

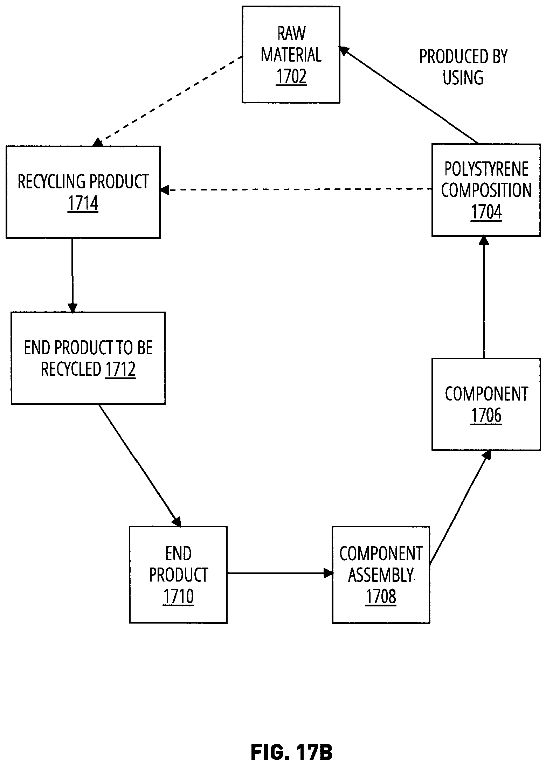

FIGs. 17A and 17B illustrate examples of relationship representations which may be used for generating a concatenation, for example the concatenation described in relation to FIGs. 11 to 16 above.

The relationship representation may relate to different stages of the chemical supply chain. The digital access elements at different stages of the chemical supply chain may be connected to the relationship representation. The relationship representation may be associated with the product produced at the respective stage of the chemical supply chain and at least one product used to produce the respective product. The relationship representation may be associated with the product produced at a respective stage of the chemical supply chain and at least one product produced at a previous stage of the chemical supply chain. The relationship representation may specify the relation between physical entities. The relationship representation may specify that the second physical entity may be used to produce the first physical entity as illustrated in Fig. 17A and/or that the first physical entity may be produced by using the second physical entity as illustrated in Fig. 17B. The relationship representation may relate to at least one precursor material used to produce the polystyrene composition. The relationship representation may relate to the polystyrene composition produced by using at least one precursor material.

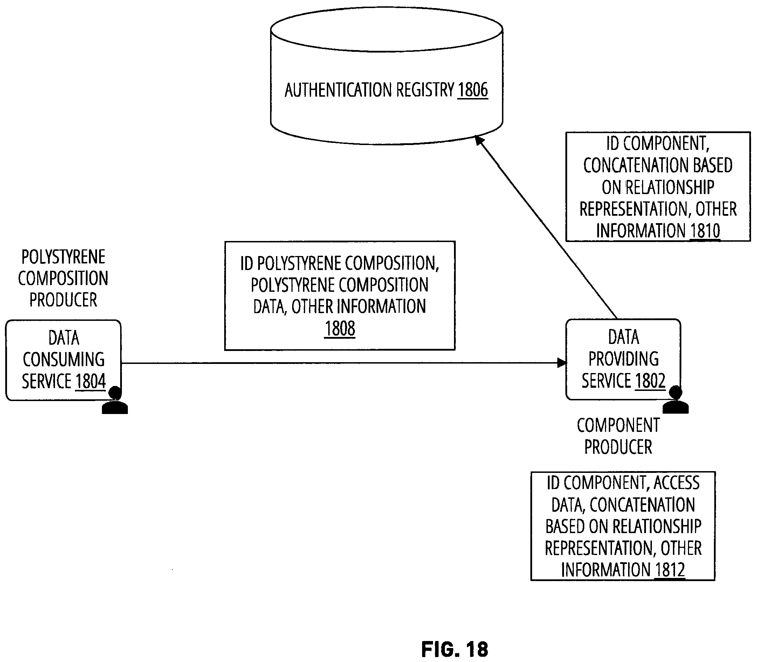

FIG. 18 illustrates an example of an apparatus for generating and using the concatenation in a decentral network. The example may be applicable to any node associated with participants of the chemical supply chain. For illustrative purposes, the example will be explained for the node associated with the polystyrene composition producer and the node associated with the component producer using the polystyrene composition to produce the component. The concatenation may be generated on generation of the component digital access element. The concatenation may be generated separately from generation of the component digital access element.

For generation of the relationship presentation, the node associated with the component producer may receive the polystyrene composition digital access element. The polystyrene composition digital access element may include or be connected to the digital identifier associated with the polystyrene composition and the access data as described in the context of FIGs. 4 to 6.

The node associated with the component producer may access the polystyrene composition data as described in the context of FIGs. 7 to 9. The node associated with the component producer may generate the component digital access element, for example as described in the context of FIGs. 3 and 6. The node associated with the component producer may generate the concatenation based on the digital identifier associated with the component, the access data associated with the component digital access element and/or the component data as well as the digital identifier associated with the polystyrene composition, the access data associated with the polystyrene composition digital access element and/or the polystyrene composition data. All variants described in the context of FIGs. 11 to 16 may be applicable

The concatenation may be provided by the component producer via the component digital access element associated with the physical entity of the produced component and/or to at least one authentication data registry (e.g. retrieval data storage). The authentication registry may store the concatenation associated with the digital identifiers. On request, the authentication registry may resolve the concatenation to track and trace physical entities of the chemical supply chain. In addition or alternatively, any node may request resolution of the concatenation generated based on data available to the nodes. Based on such relationship presentation, the nodes having access to the data required to generate the hash may resolve the concatenation to track and trace physical entities of the chemical supply chain.

The present disclosure has been described in conjunction with a preferred embodiment as examples as well. However, other variations can be understood and effected by those persons skilled in the art and practicing the claimed invention, from the studies of the drawings, this disclosure and the claims. Notably, in particular, the any steps presented can be performed in any order, i.e. the present invention is not limited to a specific order of these steps. Moreover, it is also not required that the different steps are performed at a certain place or at one node of a distributed system, i.e. each of the steps may be performed at a different nodes using different equipment/data processing units.

In the claims as well as in the description the word "comprising" does not exclude other elements or steps and the indefinite article "a" or "an" does not exclude a plurality. "can" or "may" refers to optional features. A single element or other unit may fulfill the functions of several entities or items recited in the claims. The mere fact that certain measures are recited in the mutual different dependent claims does not indicate that a combination of these measures cannot be used in an advantageous implementation.

The present disclosure has been described in conjunction with preferred embodiments and examples as well. However, other variations can be understood and effected by those persons skilled in the art and practicing the claimed invention, from the studies of the drawings, this disclosure and the claims.

Any steps presented herein can be performed in any order. The methods disclosed herein are not limited to a specific order of these steps. It is also not required that the different steps are performed at a certain place or in a certain computing node of a distributed system, i.e. each of the steps may be performed at different computing nodes using different equipment/data processing.

As used herein "determining" also includes "initiating or causing to determine", "generating" also includes "initiating and/or causing to generate" and "providing" also includes "initiating or causing to determine, generate, select, send and/or receive". "initiating or causing to perform an action" includes any processing signal that triggers a computing node or device to perform the respective action.

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation.

All terms and definitions used herein are understood broadly and have their general meaning.

## Claims

1. An apparatus for generating a digital access element associated with a polystyrene composition, wherein the apparatus comprises:
a digital identifier providing unit configured to provide a digital identifier associated with the polystyrene composition and polystyrene composition data, and
a digital access element generator configured to generate the digital access element based on the digital identifier and access data.

2. The apparatus as defined in claim 1, further comprising a physical identifier providing unit configured to provide a physical identifier for the polystyrene composition, wherein the digital identifier is associated with the physical identifier.

3. The apparatus as defined in claim 1 or 2, further comprising a collector configured to collect polystyrene composition data, wherein the digital identifier is associated with the collected polystyrene composition data.

4. The apparatus as defined in any of the preceding claims, wherein the digital identifier providing unit is configured to provide the digital identifier upon receiving a request to provide at least the digital identifier.

5. The apparatus as defined in any of the preceding claims, wherein the digital access element includes the digital identifier and the access data.

6. The apparatus as defined in any of the preceding claims, wherein the access data allow for an access to at least part of the polystyrene composition data.

7. The apparatus as defined in any of the preceding claims, wherein the digital identifier providing unit is configured to provide at least one precursor identifier associated with one or more precursor materials used to produce the polystyrene composition, and/or wherein the digital identifier unit is configured to generate a concatenation of the digital identifier associated with the polystyrene composition and the polystyrene composition data and at least one precursor identifier associated with one or more precursor materials used to produce the polystyrene composition based on a relationship representation according to which the digital identifier associated with the polystyrene composition is associated with the precursor identifier(s).

8. The apparatus as defined in claim 7, wherein the digital access element generator is configured to generate the digital access element based on the digital identifier associated with the polystyrene composition and the at least one precursor identifier or the generated concatenation.

9. The apparatus as defined in any of the preceding claims, wherein the polystyrene composition data include data on any of: one or more precursor materials used to prepare the polystyrene composition, the polystyrene composition itself.

10. An apparatus for producing a polystyrene composition, comprising the apparatus as defined in any of the preceding claims, wherein the polystyrene composition data refer to the produced polystyrene composition, and wherein the digital identifier is associated with a physical identifier of the produced polystyrene composition.

11. An apparatus for managing digital access elements for polystyrene composition s generated using the apparatus as defined in any of the preceding claims, wherein the apparatus for managing the digital access elements comprises:
a retrieval data storage for storing retrieval data associated with the digital access elements, wherein the retrieval data allow for a retrieval of stored digital access elements and/or for generating digital access elements and/or for retrieval of polystyrene composition data associated with digital access elements.

12. The apparatus as defined in claim 11, further comprising:
a digital access elements storage for storing the digital access elements, wherein the retrieval data allow for a retrieval of stored digital access elements from the digital access elements storage.

13. The apparatus as defined in claim 11 or 12, wherein the retrieval data comprise digital identifiers and associated access data of polystyrene composition s.

14. Method for generating a digital access element associated with a polystyrene composition, wherein the method includes:
providing a digital identifier associated with the polystyrene composition and polystyrene composition data, and
generating the digital access element based on the digital identifier and access data.

15. Computer program for generating a digital access element associated with polystyrene composition, wherein the program comprises instructions which, when the program is executed by one or more computers, cause the one or more computers to execute the method as defined in claim 14.
